# EUROPEAN PATENT SPECIFICATION

(11) **EP 4 701 641 B1**
(45) Date of publication and mention of the grant of the patent: **19.08.2026**
(21) Application number: 24743262.8
(22) Date of filing: 28.06.2024
(51) Int. Cl.: A61K 31/502, A61P 7/02, A61K 9/00

(54) **5-AMINO-2,3-DIHYDRO-1,4-PHTHALAZINEDIONE FOR THE PROPHYLAXIS AND TREATMENT OF THROMBOTIC DISORDERS**
5-AMINO-2,3-DIHYDRO-1,4-PHTHALAZINDIONE ZUR PROPHYLAXE UND BEHANDLUNG THROMBOTISCHER ERKRANKUNGEN
5-AMINO-2,3-DIHYDRO-1,4-PHTALAZINEDIONE POUR LA PROPHYLAXIE ET LE TRAITEMENT DE TROUBLES THROMBOTIQUES

(30) Priority: 29.06.2023 EP 23000092
(43) Date of publication of application: 04.03.2026
(73) Proprietor: MetrioPharm AG, 8021 Zürich (CH)
(72) Inventor: BRYSCH, Wolfgang, 13505 Berlin (DE); KAISER, Astrid, 12305 Berlin (DE); SAGER, Petra, 13158 Berlin (DE); SCHUMANN, Sara, 14554 Seddiner See (DE); VON WEGERER, Jörg, 13597 Berlin (DE)
(74) Representative: Gruber, Daniel
(86) International application number: PCT/EP2024/000037
(87) International publication number: WO 2025/002591

(56) References cited:
- WO-A1-2011/107295
- WO-A1-2021/249667
- SCHUMANN SARA ET AL: "Immune-Modulating Drug MP1032 with SARS-CoV-2 Antiviral Activity In Vitro: A potential Multi-Target Approach for Prevention and Early Intervention Treatment of COVID-19", vol. 21, no. 22, 20 November 2020 (2020-11-20), pages 8803, XP055781582, Retrieved from the Internet <URL:https://www.ncbi.nlm.nih.gov/pmc/articles/PMC7699954/pdf/ijms-21-08803.pdf> [retrieved on 20231201], DOI: 10.3390/ijms21228803
- GUTMANN CLEMENS ET AL: "Reactive Oxygen Species in Venous Thrombosis", vol. 21, no. 6, 11 March 2020 (2020-03-11), Basel, CH, pages 1918, XP093107484, ISSN: 1422-0067, Retrieved from the Internet <URL:https://www.ncbi.nlm.nih.gov/pmc/articles/PMC7139897/pdf/ijms-21-01918.pdf> [retrieved on 20231201], DOI: 10.3390/ijms21061918
- WEITZ JEFFREY I ET AL: "A Test in Context: D-Dimer", JOURNAL OF THE AMERICAN COLLEGE OF CARDIOLOGY, vol. 70, no. 19, 7 November 2017 (2017-11-07), pages 2411 - 2420, XP085276847, ISSN: 0735-1097, DOI: 10.1016/J.JACC.2017.09.024
- WATANABE-KUSUNOKI KANAKO ET AL: "Thrombomodulin as a Physiological Modulator of Intravascular Injury", FRONTIERS IN IMMUNOLOGY, vol. 11, 16 September 2020 (2020-09-16), Lausanne, CH, XP093107766, ISSN: 1664-3224, DOI: 10.3389/fimmu.2020.575890

## Description

The present disclosure refers to 5-amino-2,3-dihydro-1,4-phthalazinedione and its pharmaceutically acceptable salts for the prophylaxis or treatment of thrombotic disorders. The invention relates in particular to the use of 5-amino-2,3-dihydro-1,4-phthalazinedione sodium salt for this purpose. Pharmaceutical compositions, advantageous formulation techniques and a method of treatment are disclosed.

### BACKGROUND OF THE INVENTION

Thrombotic disorders are a frequent and much feared of pathological event or secondary complication of another disease. A thrombosis is a vascular disease or disorder of the circulation in which a thrombus is formed in a blood vessel. Thromboses can occur in all vessels. Most often, it is a thrombosis of the veins (venous thrombosis or phlebothrombosis), specifically a thrombosis of the deep veins of the leg (deep vein thrombosis - DVT). In pregnancy, leg vein thrombosis is a common complication. Thrombosis as a part of coagulopathy plays also a major role in sepsis.

A thrombus is formed by blood clotting - a process that basically is a protective mechanism: After an injury, the clotting system protects the body from bleeding to death. This means that the blood clots and closes the wound. This process is called hemostasis. Unlike the clotting system, the blood in the uninjured blood vessels should not clump together but flow freely. Here, a clot is a disruptive obstacle to blood flow, causes thromboembolism, and is dangerous as a trigger for pulmonary embolism. When the natural clotting ability of the blood increases abnormally, the risk of thrombosis and embolism becomes greater.

There can be congenital and exogenous factors that are in favor of thrombus formation. The so-called Virchow's triad describes the causes of the development of a venous thrombus:
- Changes in blood composition.

They can be due to coagulation disorders (hypercoagulability), hereditary increased blood coagulation, hereditary or drug-induced reduced ability to dissolve blood clots such as Activated protein C resistance (APCR), a mutation of Coagulation factor V, Protein S deficiency, foods, drugs, or toxins that affect blood clotting, such as contraceptive pills, pregnancy, dehydration, antiphospholipid syndrome.
- Diminished blood flow velocity (stasis, immobilization, hypocirculation): This can be due to dilated veins (varices) and varicose veins, limbs exposed to external pressure, immobilization caused by bed confinement (e.g., after surgery or in a cast), sitting for long periods of time with restricted movement (e.g., on a long-haul flight), presence of risk factors such as lack of exercise, overweight, desiccosis, unaccustomed heavy physical exertion, hormonal contraceptives, smoking, thrombosis in the past, pregnancy, cancer, chemotherapy, frequent intravenous injections (e.g., heroin abuse) with injury to blood vessels, above-average body size, atrial fibrillation.
- Endothelial damage

This can be due to traumas and surgeries, age-related degenerative changes, inflammatory changes of the vessels, diabetes mellitus, CO-induced hypoxic damage of the endothelium or tumor infiltrates.

Typical symptoms include swelling and a feeling of warmth combined with a feeling of tension, reddened and tense skin, possibly blue discoloration, tightness and pain in the affected limb and overheating of the swollen limb.

The most threatening complication is a thromboembolism, especially if it affects the lungs (pulmonary embolism). With a considerable percentage a thromboembolism is lethal. After myocardial infarction and stroke, a thromboembolism is the third most frequent cause of a lethal cardiovascular disease.

Recently, pulmonary embolism has gained much attention as one of the highest risks of a SARS-CoV-2 infection. For evaluating the risk of a severe disease course, the biomarker D-dimer is monitored by default. It displays a good predictability of a severe or even lethal outcome (cf. Zhan et al. (2021) Clin Appl Thrombosis Hemostasis 27: 1-10).

A prophylactic pharmaceutical treatment is mainly based on anticoagulants such as heparins (unfractioned heparin (UFH), low molecular weight heparin (LMWH), ultra-low-molecular weight heparin (ULMWH)), Vitamin K antagonists such as 4-hydroxycoumarins (warfarin, coumatetralyl, dicoumarol, phenprocoumon, brodifacoum, tioclomarol, acenocoumarol and others) and indandiones (fluindione, phenindione, pindone, chlorophacinone, diphacinone, anisindione and others).

Further pharmaceutical classes given for prophylaxis include direct thrombin inhibitors (hirudin, bivalirudin, lepirudin, desirudin, argatroban, inogatran, melagatran, ximelagatran, dabigatran, DNA aptamers, benzofuran dimers, benzofuran trimers, polymeric lignins and others) and direct factor Xa inhibitors (rivaroxaban, apixaban, edoxaban and others).

For preventing arterial thrombosis antiplatelet drugs such as ADP receptor inhibitors (clopidogrel, cangrelor, prasugrel, ticagrelor, ticlopidine and others), adenosine reuptake inhibitors (dipyridamole), glycoprotein IIB/IIIA inhibitors (abciximab, eptifibatide, tirofiban), irreversible COX inhibitors (acetylsalicylic acid, triflusal and others), PDE inhibitors (cilostazol), PAR-1 antagonists (vorapaxar) or thromboxane inhibitors (thromboxane receptor antagonists such as terutroban and thromboxane synthase inhibitors) are often given. Although antithrombotic drugs display a good prophylactic efficacy there are some problems with these medications. They are associated with adverse side effects, the most common one being an increased risk of bleeding (in particular gastrointestinal bleedings e.g., by COX inhibitors). Further not uncommon adverse side effects include skin necrosis, limb gangrene, purple toe syndrome, Vitamin K depletion and heparin-induced thrombocytopenia. Several of these drugs show unwanted interactions with other medications. Moreover, some of these drugs are very expensive (e.g., clopidogrel) which limits their use to serious cases only. A practical problem is that many of these drugs need to be administered by injection (intravenous or subcutaneous). This makes them not very popular among patients, and a purely prophylactic use (i.e., without expecting a thrombotic event) is not practical.

For thrombolysis, fibrinolytics such as streptokinase, urokinase and recombinant tissue plasminogen activators (alteplase, reteplase, tenecteplase, anistreplase) are prescribed.

Document WO2021249667 describes 5-amino-2,3-dihydro-1,4-phthalazinedione or one of its pharmaceutically acceptable salts for use in the prophylaxis or treatment of a coronaviral infection. It is further disclosed in this document that common symptoms associated with COVID-19 patients in ICU care are pulmonary embolism, thrombosis, venous thromboembolism and brain ischemia.

With regard to the SARS-CoV-2 pandemic and further viral pandemics yet to come in the future the medical need of a prophylactic (and therapeutic) medication has arisen wherein such as medication should fulfill the following profile:
- a good prophylactic effect against thrombosis
- no effect on hemostasis
- no or only negligible side effects (high safety profile)
- orally available
- ideally a drug that acts also against other causes or symptoms of the underlying disease e.g., anti-inflammatory, virostatic
- relatively low costs

Surprisingly, this task is solved by 5-amino-2,3-dihydro-1,4-phthalazinedione or its pharmaceutically acceptable salts.

Thus, the present disclosure refers to 5-amino-2,3-dihydro-1,4-phthalazinedione or one of its pharmaceutically acceptable salts, hydrates or solvates for use in the prophylaxis and treatment of a thrombotic disorder.

### DESCRIPTION OF THE INVENTION

5-amino-2,3-dihydro-1,4-phthalazinedione sodium salt was tested in a double-blinded placebo-controlled Phase II clinical study with patients undergoing medium-to-severe COVID-19. Herein, D-dimer was determined by default as a biomarker whether a thrombotic event took place or is imminent. Analysis of the taken blood samples showed that 5-amino-2,3-dihydro-1,4-phthalazinedione sodium salt reduced drastically the amount of D-dimer. This corresponded to the positive outcome of the study for 5-amino-2,3-dihydro-1,4-phthalazinedione sodium salt in combination with Standard of Care, in comparison to placebo with Standard of Care (cf. EudraCT-No.: 2021-000344-21).

This is described in Example 1.

It is known from previous studies in other indications that 5-amino-2,3-dihydro-1,4-phthalazinedione sodium salt is effective and safe in the range of 100 mg to 1000 mg daily dosage, usually split into two dosages (i.a. WO 2018/082814; WO 2022/008093; EudraCT-No.: 2014-004606-15; EudraCT-No.: 2017-003484-36). The present disclosure refers therefore to 5-amino-2,3-dihydro-1,4-phthalazinedione or one of its pharmaceutically acceptable salts, hydrates or solvates for use in the prophylaxis and treatment of a thrombotic disorder, wherein the daily dosage for a patient in need thereof is in the range of 100 mg to 1000 mg, preferred in the range of 300 mg to 800 mg, more preferred in the range of 450 mg to 700 mg.

In this clinical study no evidence was found in clinical parameters or in adverse side effects that 5-amino-2,3-dihydro-1,4-phthalazinedione sodium salt affects hemostasis. From previous *in vitro, in vivo* and clinical studies such an adverse effect is not described either. The present disclosure refers therefore to 5-amino-2,3-dihydro-1,4-phthalazinedione or one of its pharmaceutically acceptable salts, hydrates or solvates for use in the prophylaxis and treatment of a thrombotic disorder without affecting hemostasis.

In an *in vitro* cell culture model, different concentrations of 5-amino-2,3-dihydro-1,4-phthalazinedione sodium salt were added. Thereupon the amount of thrombomodulin released into the supernatant was determined. It showed that 5-amino-2,3-dihydro-1,4-phthalazinedione sodium salt increased the release of thrombomodulin significantly in a dose-dependent manner. The results are shown in Example 2.

Therefore 5-amino-2,3-dihydro-1,4-phthalazinedione sodium salt shows a clear prophylactic and therapeutic anti-thrombotic effects in these studies. It is assumed that the results in this study are predictive not only for thrombosis in COVID-19 but for other thrombotic disorders too.

5-amino-2,3-dihydro-1,4-phthalazinedione (luminol) belongs to the pharmaceutical class of the phthalazinediones. Compounds of this class are known for their beneficial anti-inflammatory action. 5-amino-2,3-dihydro-1,4-phthalazinedione is also known under the name luminol. Luminol has excellent chemiluminescent properties. It is widely applied in diagnostic assays as a detection means and in forensic medicine, for example for tracing blood spots. In medicine, 5-amino-2,3-dihydro-1,4-phthalazinedione has been developed in the form of a sodium salt. In some countries it is approved for a broad range of acute and chronic inflammatory disorders, including a.o. acute infections of bacterial and viral origin, particularly of the intestinal tract, hepatitis B and C, gastroenteritis, inflammations such as prostatitis, endometriosis, throat inflammation, bronchial asthma, pneumonia, periodontitis, pyelonephritis and autoimmune diseases such as Crohn's disease, ulcerative colitis, lupus erythematosus and scleroderma. Further, there is still a long list of indications in scientific and patent literature in the treatment of which 5-amino-2,3-dihydro-1,4-phthalazinedione sodium salt was allegedly tested or a beneficial use was suggested (cf. WO 2004/041169; WO 2007/018546; WO 2012/127441; WO 2017/202496; WO 2018/082814: a.o.).

In an *in vitro* model of SARS-CoV-2 infected cells 5-amino-2,3-dihydro-1,4-phthalazinedione sodium salt was able to suppress dose-dependently the replication of the virus (WO 2021/249667).

While most conventional immunomodulatory drugs show serious adverse reactions, or are at least problematic in long-term treatment, 5-amino-2,3-dihydro-1,4-phthalazinedione and its pharmaceutically acceptable salts are well tolerated and have a high safety margin in respect to administered dosages.

To ensure a better solubility and bioavailability pharmaceutically acceptable salts of 5-amino-2,3-dihydro-1,4-phthalazinedione are used. Sodium, potassium and lithium salts have been described for therapeutic applications (cf. WO 2010/082858). Crystal structures for lithium, sodium, potassium, rubidium and cesium salts were described in Guzei et al. (2013) Journal of Coordination Chemistry 66, 3722-3739. Thus, the present patent application refers also to the use of all pharmaceutically acceptable salts of 5-amino-2,3-dihydro-1,4-phthalazinedione.

In particular, the present application discloses 5-amino-2,3-dihydro-1,4-phthalazinedione or one of its pharmaceutically acceptable salts for use in the prophylaxis and treatment of a thrombotic disorder, wherein the pharmaceutically acceptable salt of 5-amino-2,3-dihydro-1,4-phthalazinedione is 5-amino-2,3-dihydro-1,4-phthalazinedione sodium salt.

In another aspect the present disclosure refers to 5-amino-2,3-dihydro-1,4-phthalazinedione or one of its pharmaceutically acceptable salts for use in the prophylaxis and treatment of a thrombotic disorder, wherein the prophylactic or therapeutic effect of 5-amino-2,3-dihydro-1,4-phthalazinedione or one of its pharmaceutically acceptable salts is monitored by the analysis of blood samples on their D-dimer concentration.

In a preferred embodiment said monitoring is effected by the determination of the D-dimer concentration in DDU (D-dimer units).

5-amino-2,3-dihydro-1,4-phthalazinedione is often used as a hydrate, for example as sodium salt dihydrate. Thus, the present patent application refers also to the use of all hydrates and other solvates of 5-amino-2,3-dihydro-1,4-phthalazinedione and its pharmaceutically acceptable salts. 5-amino-2,3-dihydro-1,4-phthalazinedione or one of its pharmaceutically acceptable salts may build complexes with suitable ligands. Thus, the present patent application refers also to such complexes. In the scope of the present disclosure all hydrates and solvates shall be included in the term "5-amino-2,3-dihydro-1,4-phthalazinedione or one of its pharmaceutically acceptable salts".

In order to ensure a reproducible and standardized API production and to provide improved stability features of an active agent anhydrous formulations are often preferred. Anhydrate forms of 5-amino-2,3-dihydro-1,4-phthalazinedione sodium salt have been described as crystalline polymorphs in WO 2011/107295 (Form I, Form II) and WO 2016/096143 (Form III). These crystalline polymorphs are virtually free from phase impurities and were characterized by means of X-ray powder diffraction. This method yields a set of characteristic d-values indicating interplanar spacings [Å] and of the corresponding 2-theta (2θ) angles [°] under which Bragg reflections occur. This yields a unique and unambiguous fingerprint of the respective polymorphs.

For Form I the following values were determined:
d values: 13.5; 6.9; 5.2; 4.6; 3.9; 3.5; 3.4; 3.3; 3.1; 3.0 and/or
2-theta values: 6.5; 12.7; 16.9; 19.3; 22.8; 25.8; 26.6; 27.2; 28.7; 30.3.

Form II is characterized by the following values:
d values: 12.9; 7.9; 7.1; 6.5; 5.3; 4.0; 3.7; 3.6; 3.3; 3.2 and/or
2-theta values: 6.8; 11.2; 12.5; 13.7; 16.7; 22.4; 24.3; 24.9; 27.2; 27.8.

Form III yielded the following values:
d values: 13.131; 7.987; 7.186; 6.566; 6.512; 5.372; 3.994; 3.662; 3.406; 3.288; 3.283; 3.222; 3.215; 3.127; 2.889 and/or
2-theta values: 6.73; 11.07; 12.31; 13.48; 13.59; 16.49; 22.24; 24.29; 26.14; 27.10; 27.14; 27.67; 27.72; 28.52; 30.93.

The use of anhydrous Form I of 5-amino-2,3-dihydro-1,4-phthalazinedione sodium salt is preferred.

5-amino-2,3-dihydro-1,4-phthalazinedione also shows a polymorphism. A Form I (Paradies (1992) Ber. Bunsen-Ges. Phys. Chem 96: 1027-1031) and a Form II (WO 2017/140430) have been disclosed.

Thus, the present patent application refers also to the use according to the disclosure of all crystalline forms and polymorphs thereof of 5-amino-2,3-dihydro-1,4-phthalazinedione and its pharmaceutically acceptable salts. The use of Form II of 5-amino-2,3-dihydro-1,4-phthalazinedione is preferred.

Similar therapeutic effects are known for a variety of phthalazinediones, respectively of derivatives of 5-amino-2,3-dihydro-1,4-phthalazinedione and its pharmaceutically acceptable salts. An example is 6-amino-2,3-dihydrophthalazine-1,4-dione (isoluminol). An overview of suitable phthalazinediones is given in WO 2007/018546. It is reasonable to assume that these compounds show comparable effects when being used for the therapeutic applications according to the disclosure.

Tautomerism relates to a rapid intraconversion of organic compounds in which a hydrogen atom or proton formally migrates inside the compound. This is accompanied by a switch of a single bond and adjacent double bond. The single forms are called tautomers. For example, keto-enol tautomerism occurs in 5-amino-2,3-dihydro-1,4-phthalazinedione (Proescher and Moody (1939) J Lab Clin Med, 1183-1189). Thus, the present patent application refers also to the use of all tautomers of 5-amino-2,3-dihydro-1,4-phthalazinedione and its pharmaceutically acceptable salts.

As used throughout the present application the term "5-amino-2,3-dihydro-1,4-phthalazinedione or one of its pharmaceutically acceptable salts" shall encompass all beforementioned molecular variants of 5-amino-2,3-dihydro-1,4-phthalazinedione, i.e., 5-amino-2,3-dihydro-1,4-phthalazinedione or one of its pharmaceutically acceptable salts or solvates, hydrates, crystalline polymorphs or tautomers thereof.

Unless otherwise stated, any technical or scientific term used in the present invention has the meaning that a man skilled in the relevant technical art will attribute to them.

The terms "composition" or "pharmaceutical composition" comprise at least one active ingredient in any pharmacologically acceptable defined dosage and dosage form together with at least one pharmaceutically acceptable excipient, as well as all agents that are generated from the ingredients as outlined below directly or indirectly as a combination, accumulation, complex or crystal, or as a consequence of other reactions or interactions, as well as optionally at least one further pharmaceutical drug, as listed below.

The term "excipient" is used in this application to describe any component of a pharmaceutical composition apart of the pharmaceutically active principle. The selection of suitable excipients depends on a variety of factors, such as the dosage form, the dosage, the desired solubility and the stability of the composition.

The terms "effect", "therapeutic effect", "action", "therapeutic action", "efficacy" and "effectiveness" in regard to the substance or the pharmaceutical composition of the disclosure or any other active substance mentioned in the description refers to causally occurring beneficial consequences in the organism to which said substance has been administered before.

According to the disclosure the terms "effective amount" and "therapeutically effective amount" refer to an amount of the substance of the invention that is sufficiently large to cause a desired beneficial effect in a subject in need of such a treatment.

The terms "treatment" and "therapy" comprise the administration of at least the substance of the invention, alone or in combination with at least one further pharmaceutical drug, independently of the chronological order of the administration. Such an administration is intended to substantially prevent the formation of an occlusive thrombus, respectively to improve the disease course of a thrombotic disorder.

The terms "prophylaxis" or "prophylactic treatment" comprise the administration of at least the substance of the invention, alone or in combination with at least one further pharmaceutical drug, independently of the chronological order of the administration, in order to prevent or suppress the manifestation of symptoms attributed to a thrombotic disorder. It refers in particular to medical conditions of a patient in which the manifestation of such symptoms is expected to occur in the far or near future with a reasonable probability.

The terms "subject" and "patient" comprise individuals suffering from disease symptoms or disabilities related to a thrombotic disorder wherein said diagnosis is either approved or suspected. Individuals are mammals, in particular humans.

In particular, the present application discloses 5-amino-2,3-dihydro-1,4-phthalazinedione or one of its pharmaceutically acceptable salts for use in the prophylaxis and treatment of a thrombotic disorder, wherein the thrombotic disorder is selected from a group comprising venous thrombosis, deep vein thrombosis, Paget-Schroetter disease, Budd-Chiari syndrome, portal vein thrombosis, renal vein thrombosis, cerebral venous sinus thrombosis, jugular vein thrombosis, cavernous sinus thrombosis, thrombophlebitis, perinatal thrombosis, phlegmasia alba dolens, arterial thrombosis, peripheral artery disease, acute limb ischemia, hepatic artery thrombosis, disseminated intravascular coagulation and thrombotic microangiopathy.

Venous thrombosis is a thrombosis that affects the veins of a patient.

A frequent form of venous thrombosis is deep vein thrombosis (DVT). It involves the formation of a blood clot in a deep vein, most commonly in the legs or pelvis (Phillippe (2017) Am J Managed Care 23: S376-382). Symptoms can include pain, swelling, redness, and enlarged veins in the affected area (Kruger et al. (2019) Med J Australia 210: 516-524). The greatest threat is that the thrombus detaches from the affected vein and is transported to the lungs where it causes pulmonary embolism. This can be life-threatening.

Paget-Schroetter disease is a specific form of upper extremity DVT. It typically occurs in the axillary and/or subclavian vein (Hughes (1949) Surgery, Gynecology & Obstetrics 88: 89-127).

The Budd-Chiari syndrome is a rare condition. It is caused by an occlusion of the hepatic veins that drain the liver. It is mostly due to an acquired hypercoagulability. It is often secondary to a tumor (cf. Garcia Pagán et al. (2023) New Engl J Med 388: 1307-1316).

A portal vein thrombosis is a blood clot in the hepatic portal vein. It leads to increased pressure in the portal vein and reduced blood supply to the liver (DeLeve et al. (2009) Hepatology 49: 1729-1764).

A renal vein thrombosis (RVT) is the formation of a blood clot in the vein that drains the blood from the kidneys. It can affect one or both kidneys. It mostly occurs in newly born infants with blood clotting abnormalities and adults with nephrotic syndrome (Totowa (2005) in: Handbook of Urological Emergencies: A Practical Guide; Wessels et al. eds, Humana Press, 171-180).

A cerebral venous sinus thrombosis (CVST) is a blood clot in the sinuses of the dura mater which surrounds the CNS. Symptoms may include severe headache, visual symptoms, stroke symptoms and seizures (Silvis et al. (2017) Nat Rev Neurol 13: 555-565).

A jugular vein thrombosis is often a sequela of an infection, intravenous drug use or a malignancy. Complications include systemic sepsis, pulmonary embolism and papilledema.

A cavernous sinus thrombosis (CST) is the formation of a blood clot within the cavernous sinus at the base of the brain from where deoxygenated blood is transported back to the heart. It can be caused by a spreading infection in the nose, sinuses, ears or teeth.

Thrombophlebitis is an inflammation of a vein related to a thrombus. Typical symptoms are pain of the affected area, skin redness, inflammation, edema, hardening of the veins and tenderness. One reason can be high estrogen states during pregnancy, estrogen replacement therapy or use of oral contraceptives. Other major reasons are prolonged immobility or air travel.

A perinatal thrombosis is a stroke that an infant suffers between the 140^{th} day of gestation and the 28^{th} day postpartum (Raju et al. (2007) Pediatrics 120: 609-616). Causes include birth trauma, placental abruption, infections and impaired health of the mother. In the early stages of life an affected infant may develop stroke symptoms, seizures, poor coordination and speech delays (Adén (2009) Stroke 40: 1948-1949).

Phlegmasia alba dolens includes various forms of DVT. It may occur in the third trimester of pregnancy and just after birth. It is an acute process that results in the compression of the left common iliac vein.

Arterial thrombosis is the formation of a thrombus within an artery. It is a sudden interruption of the blood flow to an organ or a body part. Symptoms include pain in the involved body part and temporarily decreased organ function.

Peripheral artery disease (PAD) is a pathologic narrowing of arteries outside those that supply the heart or the brain. It most commonly affects the legs, but also arms, neck and kidneys. A typical syndrome in the elderly is intermittent claudication.

Acute limb ischemia (ALI) is a sudden lack of blood flow to a limb within 14 days of symptoms onset (Olinic et al. (2019) J Clin Med 8 (8)). ALI is caused by embolism or thrombosis, rarely by trauma. Typical symptoms include pain, pallor, paresthesia, perishingly cold, pulselessness and paralysis (Brearly (2013) BMJ 346: f2681).

Hepatic artery thrombosis is caused by a blood clot in the artery the supplies blood to the liver. It may be a complication after liver transplantation. A major risk factor is smoking. It can lead to severe elevations in serum aminotransferases.

Disseminated intravascular coagulation (DIC) is a condition in which blood clots form throughout the body, blocking small vessels. Symptoms may include chest pain, shortness of breath, leg pain, speaking problems or immobility of certain body parts. It is a common symptom in septic disorders.

Coagulation dysfunction in septic disorders appears in the pathophysiological course of sepsis. In early stages of sepsis coagulation activation is counteracted by three anticoagulant pathways, antithrombin, protein C and tissue factor pathway inhibitor. As sepsis progresses, these three mechanisms become deranged. The result is a hypercoagulable state. Impairment of fibrinolysis contributes thus to the hypercoagulable state. It is therefore a significant therapeutic approach in sepsis treatment to restore the coagulation balance in order to stop the development of critical stages of sepsis (Tsante et al. (2023) Life 13: 350). Thrombotic microangiopathy (TMA) is a disease in which thrombosis occurs in capillaries and arterioles, due to endothelial injury. Hemolytic uremic syndrome and thrombotic thrombocytopenic purpura are subsumed hereunder.

It is preferred herein that in all the beforementioned disorders and conditions the pharmaceutically acceptable salt of 5-amino-2,3-dihydro-1,4-phthalazinedione is 5-amino-2,3-dihydro-1,4-phthalazinedione sodium salt.

The present disclosure refers thus to 5-amino-2,3-dihydro-1,4-phthalazinedione or one of its pharmaceutically acceptable salts for use in the prophylaxis or treatment of a thrombotic disorder, wherein the thrombotic disorder is selected from a group comprising venous thrombosis, deep vein thrombosis, Paget-Schroetter disease, Budd-Chiari syndrome, portal vein thrombosis, renal vein thrombosis, cerebral venous sinus thrombosis, jugular vein thrombosis, cavernous sinus thrombosis, thrombophlebitis, perinatal thrombosis, phlegmasia alba dolens, arterial thrombosis, peripheral artery disease, acute limb ischemia, hepatic artery thrombosis, disseminated intravascular coagulation, coagulation dysfunction in septic disorders and thrombotic microangiopathy.

In another aspect the present disclosure refers to 5-amino-2,3-dihydro-1,4-phthalazinedione or one of its pharmaceutically acceptable salts, hydrates or solvates for use in the prophylaxis or treatment of a thrombotic disorder, wherein the thrombotic disorder is caused by a viral infection.

In a preferred embodiment the present disclosure refers to 5-amino-2,3-dihydro-1,4-phthalazinedione or one of its pharmaceutically acceptable salts, hydrates or solvates for use in the prophylaxis or treatment of a thrombotic disorder, wherein the thrombotic disorder is caused by a SARS-CoV-2 infection.

In a preferred embodiment the present disclosure refers to 5-amino-2,3-dihydro-1,4-phthalazinedione or one of its pharmaceutically acceptable salts, hydrates or solvates for use in the prophylaxis or treatment of a thrombotic disorder, wherein the thrombotic disorder occurs during sepsis or a septic disorder.

The most common biomarker for an ongoing or imminent thrombotic disorder is the so-called D-dimer (DD). It is a degradation product physiologically occurring during fibrinolysis. A thrombus is generated when the soluble protein fibrinogen is clotted through the enzyme thrombin. The thus produced fibrin monomers thus polymerize spontaneously in a half-staggered format into protofibrils. The tensile strength of the fibrin network is enhanced by factor Xllla (a transglutaminase), which crosslinks adjacent monomers. Fibrin formation triggers also the activation of plasminogen. The thus resulting enzyme plasmin digests the individual fibrin fibers to the non-covalent trimer complex DDE. Plasmin further cleaves DDE into D-dimer (DD) and the fragment E (cf. Weitz et al. (2017) J Am Coll Cardiology 70: 2411-2420).

DD can be detected in whole blood or plasma with monoclonal antibodies that recognize an epitope on cross-linked D-dimer that is absent in the D domain of fibrinogen and non-cross-linked fibrin monomers. Three assay types are typically used: whole-blood agglutination assays, enzyme-linked immunosorbent assays (ELISA), respectively enzyme-linked immunofluorescent assays (ELFA), and latex agglutination assays (Tripodi (2011) Clin Chem 57: 1256-1262).

The enzyme thrombomodulin (TM) is encoded by the THBD gene and is predominantly expressed in the endothelium. It plays an important role in maintaining vascular homeostasis by regulating the coagulation system. Intravascular injury and inflammation lead to pro-coagulant signaling, necrotic endothelial and blood cell-derived damage-associated patterns (DAMPs) and thus mediate inflammation. During the hypercoagulable state after endothelial injury, TM is released into the intravascular space by proteolytic cleavage of endothelium components (cf. Watanabe-Kusonoki et al. (2020) Frontiers Immunol 11: 575890).

TM comprises three domain types, each with a different function: TMD1 (lectin-like domain), TMD2 (six epidermal growth factor-like structures) and TMD3 (serine- and threonine-rich domain). In response to thrombin production, TM on the endothelium acts as a thrombin receptor for reducing the coagulative properties of fibrin and platelet activation. The thrombin-TM complexes activate protein C, and the activated protein C (APC) inactivates factors Va and VIIIa, resulting in the suppression of thrombin generation. Herein TM is a natural feedback inhibitor of excessive intravascular coagulation (cf. Esmon and Owen (2004) J Thromb Haemost 2: 209-213).

To prevent unnecessary clotting, the endothelium expresses anti-coagulant factors, such as tissue factor pathway inhibitor and TM, and regulators of platelet activation, such as NO, prostacyclin, and ADPase, at steady state. In case of a vascular injury, crosstalk between the activated coagulation system and inflammatory signaling leads to mutual amplification (Foley et al. (2016) Circ Res 118: 1392-1408).

Clinical application of recombinant TM (rTM) in disseminated intravascular coagulation leads to protection from tissue injury. rTM neutralizes DAMPs, including histones and high mobility group box 1 (HMGB1), suppresses excessive activation of the complement system, protects the endothelium and influences both the innate and acquired immune system. Neutrophil extracellular traps (NETs) promote immunothrombosis by orchestrating platelets to enclose infectious invaders as part of the innate immune system. Excessive immunothrombosis, however, can cause intravascular injury (cf. Watanabe-Kusonoki et al. (2020) Frontiers Immunol 11: 575890). The immunothrombotic clot consists basically of fibrin. rTM inhibits NET formation following treatment with LPS-primed platelets by suppressing TLR4 signaling (Shimomura et al. (2016) J Intensive Care 4: 48). Upon binding to neutrophils autoantibody-mediated NET formation is inhibited (Watanabe-Kusoniki et al. (2020) J Autoimmun 108: 102390).

It can be reasonably assumed that by pharmaceutically enhancing the TM levels in blood the same effects can be triggered as by applying rTM. This is shown in Example 2.

In another aspect the present disclosure refers to a pharmaceutical composition containing 5-amino-2,3-dihydro-1,4-phthalazinedione or one of its pharmaceutically acceptable salts, hydrates or solvates and at least one pharmaceutically acceptable excipient for use in the in the prophylaxis or treatment of a thrombotic disorder. It is preferred that said pharmaceutically acceptable salt is 5-amino-2,3-dihydro-1,4-phthalazinedione sodium salt.

In another aspect the present disclosure refers to said pharmaceutical composition for use in the prophylaxis or treatment of a thrombotic disorder, wherein said pharmaceutical composition is suitable for inhalatory, oral, parenteral, intraperitoneal, intravenous, intraarterial, intramuscular, topical, transdermal, subcutaneous, intradermal, sublingual, conjunctival, intravaginal, rectal, intrathecal, pharyngeal or nasal administration or by intubation.

The term "pharmaceutically acceptable excipient(s)" refers to natural or synthetic compounds that are added to a pharmaceutical formulation alongside the pharmaceutical active agent. They may help to bulk up the formulation, to enhance the desired pharmacokinetic properties or the stability of the formulation, as well as being beneficial in the manufacturing process.

In another aspect the present disclosure refers to said pharmaceutical compositions for use in the in the prophylaxis or treatment of a thrombotic disorder, wherein said at least one pharmaceutically acceptable excipient is selected from a group comprising carriers, binding agents, colorants, buffers, preservatives, antioxidants, coatings, sweeteners, thickening agents, pH-regulators, acidity regulators, acidifiers, solvents, isotonizing agents, disintegrants, glidants, lubricants, emulsifiers, solubilizing agents, stabilizers, diluents, anti-caking agents, sorbents, permeation enhancers, foaming agents, anti-foaming agents, opacifiers, fatliquors, consistency enhancers, hydrotropes, aromatic and flavoring substances.

In general, one or more pharmaceutically acceptable carriers are added to a pharmaceutically active agent. Eligible are all carriers known in the art and combinations thereof. In solid dosage forms they can be for example plant and animal fats, waxes, paraffins, starch, tragacanth, cellulose derivatives, polyethylene glycols, silicones, bentonites, silica, talcum, zinc oxide. For liquid dosage forms and emulsions suitable carriers are for example solvents, solubilizing agents, emulsifiers such as water, ethanol, isopropanol, ethyl carbonate, ethyl acetate, benzyl alcohol, benzyl benzoate, propylene glycol, 1,3-butyl glycol, cotton seed oil, peanut oil, olive oil, castor oil, sesame oil, glycerol fatty acid esters, polyethylglycols, fatty acid esters of sorbitan. Suspensions according to the disclosure may use carriers known in the art such as diluents (e.g., water, ethanol or propylene glycol), ethoxylated isostearyl alcohols, polyoxyethylene and polyoxyethylene sorbitan esters, microcrystalline cellulose, bentonites, agar agar, tragacanth.

The term binding agents refers to substances that bind powders or glue them together, rendering them cohesive through granule formation. They serve as a "glue" of the formulation. Binding agents increase the cohesive strength of the provided diluent or filler.

Suitable binding agents are for example starch from wheat, corn, rice or potato, gelatin, naturally occurring sugars such as glucose, sucrose or beta-lactose, sweeteners from corn, natural and synthetic gums such as acacia, tragacanth or ammonium calcium alginate, sodium alginate, carboxymethyl cellulose, sodium carboxymethyl cellulose, hydroxypropyl carboxymethyl cellulose, polyethylene glycol, polyvinyl pyrrolidone, magnesium aluminum silicate, waxes and others. The percentage of the binding agent in the composition can range from 1 - 30 % by weight, preferred 2 - 20 % by weight, more preferred 3 - 10 % by weight and most preferred 3 - 6 % by weight.

Colorants are excipients that bestow a colorization to the pharmaceutical formulation. These excipients can be food colorants. They can be adsorbed on a suitable adsorption means such as clay or aluminum oxide. A further advantage of a colorant is that it may visualize spilled aqueous solution on the nebulizer and/or the mouthpiece to facilitate cleaning. The amount of the colorant may vary between 0.01 and 10% per weight of the pharmaceutical composition, preferred between 0.05 and 6 % per weight, more preferred between 0.1 and 4 % per weight, most preferred between 0.1 and 1 % per weight.

Suitable pharmaceutical colorants are for example curcumin, riboflavin, riboflavin-5'-phosphate, tartrazine, alkannin, quinolione yellow WS, Fast Yellow AB, riboflavin-5'-sodium phosphate, yellow 2G, Sunset yellow FCF, orange GGN, cochineal, carminic acid, citrus red 2, carmoisine, amaranth, Ponceau 4R, Ponceau SX, Ponceau 6R, erythrosine, red 2G, Allura red AC, Indanthrene blue RS, Patent blue V, indigo carmine, Brilliant blue FCF, chlorophylls and chlorophyllins, copper complexes of chlorophylls and chlorophyllins, Green S, Fast Green FCF, Plain caramel, Caustic sulphite caramel, ammonia caramel, sulphite ammonia caramel, Black PN, Carbon black, vegetable carbon, Brown FK, Brown HT, alpha-carotene, beta-carotene, gamma-carotene, annatto, bixin, norbixin, paprika oleoresin, capsanthin, capsorubin, lycopene, beta-apo-8'-carotenal, ethyl ester of beta-apo-8'-carotenic acid, flavoxanthin, lutein, cryptoxanthin, rubixanthin, violaxanthin, rhodoxanthin, canthaxanthin, zeaxanthin, citranaxanthin, astaxanthin, betanin, anthocyanins, saffron, calcium carbonate, titanium dioxide, iron oxides, iron hydroxides, aluminum, silver, gold, pigment rubine, tannin, orcein, ferrous gluconate, ferrous lactate.

Moreover, buffer solutions are preferred for liquid formulations, in particular for pharmaceutical liquid formulations. The terms buffer, buffer system and buffer solution, in particular of an aqueous solution, refer to the capacity of the system to resist a pH change by the addition of an acid or a base, or by dilution with a solvent. Preferred buffer systems may be selected from the group comprising formate, lactate, benzoic acid, oxalate, fumarate, aniline, acetate buffer, citrate buffer, glutamate buffer, phosphate buffer, succinate, pyridine, phthalate, histidine, MES (2-(N-morpholino) ethanesulfonic acid), maleic acid, cacodylate (dimethyl arsenate), carbonic acid, ADA (N-(2-acetamido)imino diacetic acid, PIPES (4-piperazine-bis-ethanesulfonic acid), BIS-TRIS propane (1,3-bis[tris(hydroxymethyl)methylamino] propane), ethylene diamine, ACES (2-[(amino-2-oxoethyl)amino]ethanesulfonic acid), imidazole, MOPS (3-(N-morpholino) propanesulfonic acid), diethyl malonic acid, TES (2-[tris(hydroxymethyl)methyl]aminoethanesulfonic acid), HEPES (*N*-2-hydroxyethylpiperazine-N'-2-ethanesulfonic acid), as well as other buffers with a pKₐ between 3.8 and 7.7.

Preferred are carbonic acid buffers such as acetate buffer and dicarboxylic acid buffers such as fumarate, tartrate and phthalate as well as tricarboxylic acid buffers such as citrate.

A further group of preferred buffers are inorganic buffers such as sulfate hydroxide, borate hydroxide, carbonate hydroxide, oxalate hydroxide, calcium hydroxide and phosphate buffers. Another group of preferred buffers are nitrogen-containing puffers such as imidazole, diethylene diamine and piperazine. Furthermore preferred are sulfonic acid buffers such as TES, HEPES, ACES, PIPES, [(2-hydroxy-1,1-bis-(hydroxymethyl)ethyl)amino]-1-propanesulfonic acid (TAPS), 4-(2-hydroxyethyl)piperazine-1-propanesulfonic acid (EEPS), MOPS and N,N-bis-(2-hydroxyethyl)-2-aminoethanesulfonic acid (BES). Another group of preferred buffers are glycine, glycyl-glycine, glycyl-glycyl-glycine, N,N-bis-(2-hydroxyethyl)glycine and N-[2-hydroxy-1,1-bis(hydroxymethyl)ethyl]glycine (tricine). Preferred are also amino acid buffers such as glycine, alanine, valine, leucine, isoleucine, serine, threonine, phenylalanine, tyrosine, tryptophan, lysine, arginine, histidine, aspartate, glutamate, asparagine, glutamine, cysteine, methionine, proline, 4-hydroxy proline, *N,N,N-*trimethyllysine, 3-methyl histidine, 5-hydroxy lysine, o-phosphoserine, gamma-carboxyglutamate, [epsilon]-N-acetyl lysine, [omega]-N-methyl arginine, citrulline, ornithine and their derivatives. Particularly preferred is KH₂PO₄ buffer.

Preservatives for liquid and/or solid dosage forms can be used on demand. They may be selected from the group comprising, but not limited to, sorbic acid, potassium sorbate, sodium sorbate, calcium sorbate, methyl paraben, ethyl paraben, methyl ethyl paraben, propyl paraben, benzoic acid, sodium benzoate, potassium benzoate, calcium benzoate, heptyl p-hydroxybenzoate, sodium methyl para-hydroxybenzoate, sodium ethyl para-hydroxybenzoate, sodium propyl para-hydroxybenzoate, benzyl alcohol, benzalkonium chloride, phenylethyl alcohols, cresols, cetylpyridinium chloride, chlorobutanol, thiomersal (sodium 2-(ethylmercurithio) benzoic acid), sulfur dioxide, sodium sulfite, sodium bisulfite, sodium metabisulfite, potassium metabisulfite, potassium sulfite, calcium sulfite, calcium hydrogen sulfite, potassium hydrogen sulfite, biphenyl, orthophenyl phenol, sodium orthophenyl phenol, thiabendazole, nisin, natamycin, formic acid, sodium formate, calcium formate, hexamine, formaldehyde, dimethyl dicarbonate, potassium nitrite, sodium nitrite, sodium nitrate, potassium nitrate, acetic acid, potassium acetate, sodium acetate, sodium diacetate, calcium acetate, ammonium acetate, dehydroacetic acid, sodium dehydroacetate, lactic acid, propionic acid, sodium propionate, calcium propionate, potassium propionate, boric acid, sodium tetraborate, carbon dioxide, malic acid, fumaric acid, lysozyme, copper-(II)-sulfate, chlorine, chlorine dioxide and other suitable substances or compositions known to the person skilled in the art.

The addition of a sufficient amount of antioxidants is particularly preferable for liquid and topical dosage forms. Suitable examples for antioxidants include sodium metabisulfite, alpha-tocopherol, ascorbic acid, maleic acid, sodium ascorbate, ascorbyl palmitate, butylated hydroxyanisol, butylated hydroxytoluene, fumaric acid or propyl gallate. Preferred is the use of sodium metabisulfite, alpha-tocopherol and ascorbyl palmitate.

Tablets or pills are usually coated i.e., the coating constitutes the outer layer. This can be a film coating, a sugar coating with saccharides and a compression coating. Pharmaceutically acceptable varnishes or waxes, HPMC (hydroxypropylmethylcellulose), MC (methylcellulose) or HPC (hydroxypropylcellulose) can be used. Such a coating may help to disguise the taste, to ease the swallowing or the identification. Often plasticizers and pigments are included in the coating. Capsules normally have a gelatinous envelope that encloses the active substance(s). The specific composition and thickness of this gelatinous layer determines how fast absorption takes place after ingestion of the capsule. Of special interest are sustained release formulations, as known in the art.

Suitable sweeteners can be selected from the group comprising mannitol, glycerol, acesulfame potassium, aspartame, cyclamate, isomalt, isomaltitol, saccharin and its sodium, potassium and calcium salts, sucralose, alitame, thaumatin, glycyrrhizin, neohesperidine dihydrochalcone, steviol glycosides, neotame, aspartame-acesulfame salt, maltitol, maltitol syrup, lactitol, xylitol, erythritol.

Suitable thickening agents can be selected from the group comprising, but not limited to, polyvinyl pyrrolidone, methyl cellulose, hydroxypropyl methyl cellulose, hydroxypropyl cellulose, dextrins, polydextrose, modified starch, alkaline modified starch, bleached starch, oxidized starch, enzyme-treated starch, monostarch phosphate, distarch phosphate esterified with sodium trimetaphosphate or phosphorus oxychloride, phosphate distarch phosphate, acetylated distarch phosphate, starch acetate esterified with acetic anhydride, starch acetate esterified with vinyl acetate, acetylated distarch adipate, acetylated distarch glycerol, distarch glycerin, hydroxypropyl starch, hydroxy propyl distarch glycerin, hydroxypropyl distarch phosphate, hydroxypropyl distarch glycerol, starch sodium octenyl succinate, acetylated oxidized starch, hydroxyethyl cellulose.

Suitable pH-regulators for liquid dosage forms are e.g., sodium hydroxide, hydrochloric acid, buffer substances such as sodium dihydrogen phosphate or disodium hydrogenphosphate.

Suitable acidity regulators can be selected from the group comprising acetic acid, potassium acetate, sodium acetate, sodium diacetate, calcium acetate, carbon dioxide, malic acid, fumaric acid, sodium lactate, potassium lactate, calcium lactate, ammonium lactate, magnesium lactate, citric acid, mono-, di-, trisodium citrate, mono-, di-, tripotassium citrate, mono-, di-, tricalcium citrate, tartaric acid, mono-, disodium tartrate, mono-, dipotassium tartrate, sodium potassium tartrate, ortho-phosphoric acid, lecithin citrate, magnesium citrate, ammonium malate, sodium malate, sodium hydrogen malate, calcium malate, calcium hydrogen malate, adipic acid, sodium adipate, potassium adipate, ammonium adipate, succinic acid, sodium fumarate, potassium fumarate, calcium fumarate, ammonium fumarate, 1,4-heptonolactone, triammonium citrate, ammonium ferric citrate, calcium glycerophosphate, isopropyl citrate, potassium carbonate, potassium bicarbonate, ammonium carbonate, ammonium bicarbonate, magnesium carbonate, magnesium bicarbonate, ferrous carbonate, ammonium sulfate, aluminum potassium sulfate, aluminum ammonium sulfate, sodium hydroxide, potassium hydroxide, ammonium hydroxide, magnesium hydroxide, gluconic acid.

Acidifiers use to be inorganic chemicals that either produce or become acid. Suitable examples are ammonium chloride and calcium chloride.

Suitable solvents may be selected from the group comprising, but not limited to, water, carbonated water, water for injection, water with isotonizing agents, saline, isotonic saline, alcohols, particularly ethyl and n-butyl alcohol, and mixtures thereof.

Suitable isotonizing agents are for example pharmaceutically acceptable salts, in particular sodium chloride and potassium chloride, sugars such as glucose or lactose, sugar alcohols such as mannitol and sorbitol, citrate, phosphate, borate and mixtures thereof.

Suitable disintegrants can be selected from the group comprising starch, cold water-soluble starches such as carboxymethyl starch, cellulose derivatives such as methyl cellulose and sodium carboxymethyl cellulose, microcrystalline cellulose and cross-linked microcrystalline celluloses such as croscarmellose sodium, natural and synthetic gums such as guar, agar, karaya (Indian tragacanth), locust bean gum, tragacanth, clays such as bentonite, xanthan gum, alginates such as alginic acid and sodium alginate, foaming compositions i.a. Moisture expansion is supported by for example starch, cellulose derivatives, alginates, polysaccharides, dextrans, cross-linked polyvinyl pyrrolidone. The amount of the disintegrant in the composition may vary between 1 and 40% per weight, preferred between 3 and 20% per weight, most preferred between 5 and 10% per weight.

Glidants are materials that prevent a baking of the respective supplements and improve the flow characteristics of granulations so that the flow is smooth and constant. Suitable glidants comprise silicon dioxide, magnesium stearate, sodium stearate, starch and talcum. The amount of the glidant in the composition may vary between 0.01 and 10% per weight, preferred between 0.1 and 7% per weight, more preferred between 0.2 and 5% per weight, most preferred between 0.5 and 2% per weight.

The term "lubricants" refers to substances that are added to the dosage form in order to facilitate tablets, granulates etc. to be released from the press mold or the outlet nozzle. They diminish friction or abrasion. Lubricants are usually added shortly before pressing, as they should be present on the surface of the granules and between them and the parts of the press mold. The amount of the lubricant in the composition may vary between 0.05 and 15% per weight, preferred between 0.2 and 5% per weight, more preferred between 0.3 and 3% per weight, most preferred between 0.3 and 1.5% per weight. Suitable lubricants are a.o. sodium oleate, metal stearates such as sodium stearate, calcium stearate, potassium stearate and magnesium stearate, stearic acid, sodium benzoate, sodium acetate, sodium chloride, boric acid, waxes having a high melting point, polyethylene glycol.

Emulsifiers can be selected for example from the following anionic and non-ionic emulsifiers: Anionic emulsifier waxes, cetyl alcohol, cetylstearyl alcohol, stearic acid, oleic acid, polyoxyethylene polyoxypropylene block polymers, addition products of 2 to 60 mol ethylene oxide to castor oil and/or hardened castor oil, wool wax oil (lanolin), sorbitan esters, polyoxyethylene alkyl esters, polyoxyethylene sorbitan fatty acid esters, polyoxyethene sorbitan monolaurate, polyoxyethene sorbitan monooleate, polyoxyethene sorbitan monopalmitate, polyoxyethene sorbitan monostearate, polyoxyethene sorbitan tristearate, polyoxyethene stearate, polyvinyl alcohol, metatartaric acid, calcium tartrate, alginic acid, sodium alginate, potassium alginate, ammonium alginate, calcium alginate, propane-1,2-diol alginate, carrageenan, processed Eucheuma seaweed, locust bean gum, tragacanth, acacia gum, karaya gum, gellan gum, gum ghatti, glucomannan, pectin, amidated pectin, ammonium phosphatides, brominated vegetable oil, sucrose acetate isobutyrate, glycerol esters of wood rosins, disodium phosphate, trisodium diphosphate, tetrasodium diphosphate, dicalcium diphosphate, calcium dihydrogen diphosphate, sodium triphosphate, pentapotassium triphosphate, sodium polyphosphates, sodium calcium polyphosphate, calcium polyphosphates, ammonium polyphosphate, beta-cyclodextrin, powdered cellulose, methyl cellulose, ethyl cellulose, hydroxypropyl cellulose, hydroxypropyl methylcellulose, ethyl methyl cellulose, carboxymethyl cellulose, sodium carboxymethyl cellulose, ethyl hydroxyethyl cellulose, croscarmellose, enzymically hydrolyzed carboxymethyl cellulose, mono- and diglycerides of fatty acids, glyceryl monostearate, glyceryl distearate, acetic acid esters of mono- and diglycerides of fatty acids, lactic acid esters of mono- and diglycerides of fatty acids, citric acid esters of mono- and diglycerides of fatty acids, tartaric acid esters of mono- and diglycerides of fatty acids, mono- and diacetyl tartaric acid esters of mono- and diglycerides of fatty acids, mixed acetic and tartaric acid esters of mono- and diglycerides of fatty acids, succinylated monoglycerides, sucrose esters of fatty acids, sucroglycerides, polyglycerol esters of fatty acids, polyglycerol polyricinoleate, propane-1,2-diol esters of fatty acids, propylene glycol esters of fatty acids, lactylated fatty acid esters of glycerol and propane-1, thermally oxidized soy bean oil interacted with mono- and diglycerides of fatty acids, dioctyl sodium sulphosuccinate, sodium stearoyl-2-lactylate, calcium stearoyl-2-lactylate, stearyl tartrate, stearyl citrate, sodium stearoyl fumarate, calcium stearoyl fumarate, stearyl tartrate, stearyl citrate, sodium stearoyl fumarate, calcium stearoyl fumarate, sodium laurylsulfate, ethoxylated mono- and diglycerides, methyl glucoside-coconut oil ester, sorbitan monostearate, sorbitan tristearate, sorbitan monolaurate, sorbitan monooleate, sorbitan monopalmitate, sorbitan trioleate, calcium sodium polyphosphate, calcium polyphosphate, ammonium polyphosphate, cholic acid, choline salts, distarch glycerol, starch sodium octenyl succinate, acetylated oxidized starch. Preferred are glycerin monooleate, stearic acid, phospholipids such as lecithin.

Suitable as surface-active solubilizing agents (solubilizers) are for example diethylene glycol monoethyl ester, polyethyl propylene glycol co-polymers, cyclodextrins such as α- and β-cyclodextrin, glyceryl monostearates such as Solutol HS 15 (Macrogol-15-hydroxystearate from BASF, PEG 660-15 hydroxystearates), sorbitan esters, polyoxyethylene glycol, polyoxyethylene sorbitanic acid esters, polyoxyethylene sorbitan monooleate, polyoxyethylene oxystearic acid triglyceride, polyvinyl alcohol, sodium dodecyl sulfate, (anionic) glyceryl monooleates.

Stabilizers are substances that can be added to prevent unwanted changes. Though stabilizers are not real emulsifiers they may also contribute to the stability of emulsions. Suitable examples for stabilizers are oxystearin, xanthan gum, agar, oat gum, guar gum, tara gum, polyoxyethene stearate, aspartame-acesulfame salt, amylase, proteases, papain, bromelain, ficin, invertase, polydextrose, polyvinyl pyrrolidone, polyvinyl polypyrrolidone, triethyl citrate, maltitol, maltitol syrup.

Diluents or fillers are inactive substances added to drugs for handling minimal amounts of active agents. Examples for suitable diluents are water, mannitol, pre-gelatinized starch, starch, microcrystalline cellulose, powdered cellulose, silicified microcrystalline cellulose, dibasic calcium phosphate dihydrate, calcium phosphate, calcium carbonate, hydroxypropyl cellulose, hydroxyethyl cellulose, hydroxypropyl methylcellulose, polyethylene glycol, xanthan gum, gum arabic or any combination thereof.

Anti-caking agents (antiadherents) can be added to a supplement or a composition of supplements for preventing the formation of lumps and for easing packaging, transport, release from the at least one chamber of the dispensing cap and consumption. Suitable examples include tricalcium phosphate, powdered cellulose, magnesium stearate, sodium bicarbonate, sodium ferrocyanide, potassium ferrocyanide, calcium ferrocyanide, bone phosphate, sodium silicate, silicon dioxide, calcium silicate, magnesium trisilicate, talcum powder, sodium aluminosilicate, potassium aluminum silicate, calcium aluminosilicate, bentonite, aluminum silicate, stearic acid, polydimethyl siloxane.

Sorbents are materials that soak up oil from the water. Suitable examples include natural sorbents such as peat moss, sawdust, feathers, and anything else natural that contains carbon and synthetic sorbents such as polyethylene and nylon. Sorbents are used for tablet/capsule moisture-proofing by limited fluid sorbing (taking up of a liquid or a gas either by adsorption or by adsorption) in a dry state.

Permeation enhancers are often used in topical dosage forms. Suitable permeation enhancers comprise all pharmaceutically acceptable permeation enhancers known in the art, such as, without being limiting, azones such as laurocapram, 1-dodecylazacycloheptan-2-one; sulfoxides such as dimethyl sulfoxide, DMAC, DMF; pyrrolidones such as 2-pyrrolidone, N-methyl-2-pyrrolidone; alcohols such as ethanol, 1,2-propandiol or decanol; glycols such as propylene glycol, diethylene glycol, tetraethylene glycol; fatty acids such as oleic acid, lauric acid, sodium lauryl sulfate, myristic acid, isopropyl myristic acid, capric acid; nonic surfactants such as polyoxyethylene-2-oleyl ether, polyoxyethylene-2-stearyl ether; terpenes; terpenoids; oxazolidinones; urea; ceramide analogs, azone analogs, menthol derivatives, etherified derivatives, esterified derivatives, transcarbams, carbamate salts, TXA derivatives, DDAIP (dodecyl 2-(dimethylamino) propanoate), DDAK, natural essential oils (all of them listed in Chen et al. (2014) Asian J. Pharm. Sc. 9, 51-64); citric acid esters such as triethyl citrate; hydrophobin polypeptides; alpha-bisabolol; dimethyl isosorbide (Arlasove^{®} DMI); ethoxydiglycol. Preferred is 1,2-propandiol.

In some galenic formulations it may be desirable that a liquid oral dosage form generates some foam on being dissolved. Such an effect can be supported through the addition of a foaming agent that reduces the surface tension of the liquid, thus facilitating the formation of bubbles, or it increases its colloidal stability by inhibiting coalescence of bubbles. Alternatively, it may stabilize foam. Suitable examples include mineral oil, quillaia extract, triethyl citrate, sodium lauryl ether sulfate, sodium lauryl sulfate, ammonium lauryl sulfate.

Alternatively, some liquid oral dosage forms may appear slightly foamy upon preparation. Though this does not interfere with the desired application it may affect patient compliance in case of a medication or the commercial success in case of dietary supplements. Therefore, it may be desirable to add a pharmaceutically acceptable anti-foaming agent (defoamer). Examples are polydimethylsiloxane or silicone oil in dietary supplements or simethicone in pharmaceuticals.

Opacifiers are substances that render the liquid dosage for, opaque, if desired. They must have a refractive index substantially different from the solvent, in most cases here water. At the same time, they should be inert to the other components of the composition. Suitable examples include titanium dioxide, talc, calcium carbonate, behenic acid, cetyl alcohol, or mixtures thereof.

Suitable fatliquors are e.g., oleic acid decyl ester, hydrated castor oil, light mineral oil, mineral oil, polyethylene glycol, sodium laurylsulfate.

Consistency enhancers are e.g., cetyl alcohol, cetyl ester wax, hydrated castor oil, microcrystalline waxes, non-ionic emulsifier waxes, beeswax, paraffin or stearyl alcohol.

Suitable hydrotropes are alcohols such as ethanol, isopropyl alcohol or polyols such as glycerin.

Suitable aromatic and flavoring substances comprise above all essential oils that can be used for this purpose. In general, this term refers to volatile extracts from plants or parts of plants with the respective characteristic smell. They can be extracted from plants or parts of plants by steam distillation.

Suitable examples are: Essential oils, respectively aromatic substances from achillea, sage, cedar, clove, chamomile, anise, aniseed, star anise, thyme, tea tree, peppermint, mint oil, menthol, cineol, borneol, zingerol, eucalyptus, mango, figs, lavender oil, chamomile blossoms, pine needle, cypress, orange, rose, rosewood, plum, currant, cherry, birch leaves, cinnamon, lime, grapefruit, tangerine, juniper, valerian, lemon, lemon balm, lemon grass, palmarosa, cranberry, pomegranate, rosemary, ginger, pineapple, guava, echinacea, ivy leave extract, blueberry, kaki, melon, alpha- or beta-pinene, alpha-pinene oxide, alpha-campholenic aldehyde, alpha-citronellol, alpha-isoamyl-cinnamic, alpha-cinnamic terpinene, alpha-terpineol, alpha-terpinene, aldehyde C₁₆, alpha-phellandrene, amyl cinnamic aldehyde, amyl salicylate, anisic aldehyde, basil, anethole, bay, benzyl acetate, benzyl alcohol, bergamot, bitter orange peel, black pepper, calamus, camphor, cananga oil, cardamom, carnation, carvacrol, carveol, cassia, castor, cedarwood, cinnamaldehyde, cinnamic alcohol, cis-pinane, citral, citronella, citronellal, citronellol dextro, citronellol, citronellyl acetate; citronellyl nitrile, citrus unshiu, clary sage, clove bud, coriander, corn, cotton seed, d-dihydrocarvone, decyl aldehyde, diethyl phthalate, dihydroanethole, dihydrocarveol, dihydrolinalool, dihydromyrcene, dihydromyrcenol, dihydromyrcenyl acetate; dihydroterpineol, dimethyl salicylate, dimethyloctanal, dimethyloctanol, dimethyloctanyl acetate, diphenyl oxide, dipropylene glycol, d-limonene, d-pulegone, estragole, ethyl vanillin, eucalyptol; eucalyptus citriodora, eucalyptus globulus, eugenol, evening primrose, fenchol, fennel, ferniol, fish, florazon, galaxolide, geraniol, geranium, geranyl acetate, geranyl nitrile, guaiacol, guaiacwood, gurjun balsam, heliotropin, herbanate, hiba, hydroxycitronellal, i-carvone, i-methyl acetate, ionone, isobutyl quinoleine, isobornyl acetate, isobornyl methylether, isoeugenol, isolongifolene, jasmine, lavender, limonene, linallol oxide, linallol, linalool, linalyl acetate, linseed, litsea cubeba, I-methyl acetate, longifolene, mandarin, mentha, menthane hydroperoxide, menthol crystals, menthol laevo, menthone laevo, methyl anthranilate, methyl cedryl ketone, methyl chavicol, methyl hexyl ether, methyl ionone, methyl salicylate, mineral, mint, musk ambrette, musk ketone, musk xylol, myrcene, nerol, neryl acetate, nonyl aldehyde, nutmeg, orris root, para-cymene, parahydroxy phenyl butanone crystals, patchouli, p-cymene, pennyroyal oil, pepper, perillaldehyde, petitgrain, phenyl ethyl alcohol, phenyl ethyl propionate, phenyl ethyl-2methylbutyrate, pimento berry, pimento leaf, pinane hydroperoxide, pinanol, pine ester, pine, pinene, piperonal, piperonyl acetate, piperonyl alcohol, plinol, plinyl acetate, pseudo ionone, rhodinol, rhodinyl acetate, rosalin, ryu, sandalwood, sandenol, sassafras, sesame, soybean, spearmint, spice, spike lavender, spirantol, starflower, tea seed, terpenoid, terpineol, terpinolene, terpinyl acetate, tert-butylcyclohexyl acetate, tetrahydrolinalool, tetrahydrolinalyl acetate, tetrahydromyrcenol, thulasi, thymol, tomato, trans-2-hexenol, trans-anethole, turmeric, turpentine, vanillin, vetiver, vitalizair, white cedar, white grapefruit, wintergreen etc. or mixtures thereof, as well as mixtures of menthol, peppermint and star anise oil or menthol and cherry flavor.

These aromatic or flavoring substances can be included in the range of 0.0001 to 10 % per weight (particularly in a composition), preferred 0.001 to 6% per weight, more preferred 0.001 to 4% per weight, most preferred 0.01 to 1% per weight, with regard to the total composition. Application- or single case-related it may be advantageous to use differing quantities.

According to the disclosure all the beforementioned excipients and classes of excipients can be used without limitation alone or in any conceivable combination thereof, as long as the inventive use is not thwarted, toxic actions may occur, or respective national legislations are infracted.

In another aspect of the invention the present application relates to 5-amino-2,3-dihydro-1,4-phthalazinedione or one of its pharmaceutically acceptable salts or a pharmaceutical composition according to the disclosure for use in a formulation for oral administration in the prophylaxis or treatment of a thrombotic disorder.

Pharmaceutical formulations suitable for oral dosage forms of 5-amino-2,3-dihydro-1,4-phthalazinedione or one of its pharmaceutically acceptable salts or a pharmaceutical composition according to the disclosure may be administered as separate units such as tablets, soft gelatin capsules, hard gelatin capsules, sugar-coated tablets or pills; powders or granulates; juices, syrups, drops, teas, solutions or suspensions in aqueous or non-aqueous liquids; edible foams or mousses; or in oil-in-water or water-in-oil in emulsions.

In oral dosage forms such as tablets or capsules the active agent can thus be combined with a non-toxic and pharmaceutically acceptable inert carrier such as ethanol, glycerol or water. Powders are produced by grinding the compound to a suitably tiny particle size and mixing them with a pharmaceutical carrier in a similar manner e.g., an edible carbohydrate such as starch or mannitol. A flavor, preservative, dispersant or colorant can also be present.

Tablets are formulated by producing, granulating or dry pressing a powder mixture, adding a lubricant and a disintegrants and pressing the mixture to a tablet. A powder mixture is produced by mixing a suitably ground compound with a diluent or a base as described before, and if applicable, with a binding agent such as carboxymethyl cellulose, an alginate, gelatin or polyvinyl pyrrolidone, a dissolution retardant, such as, for example, paraffin, an absorption accelerator, such as, for example, a quaternary salt, and/or an absorbent, such as, for example, bentonite, kaolin or dicalcium phosphate. The powder mixture can be granulated by wetting it with a binder, such as, for example, syrup, starch paste, acacia mucilage or solutions of cellulose or polymer materials and pressing it through a sieve. As an alternative to granulation, the powder mixture can be run through a tableting machine, giving lumps of non-uniform shape which are broken up to form granules. The granules can be lubricated by addition of stearic acid, a stearate salt, talc or mineral oil in order to prevent sticking to the tablet casting mold. The lubricated mixture is then pressed to give tablets. The compounds according to the disclosure can also be combined with a free-flowing inert excipient and then pressed directly to give tablets without carrying out the granulation or dry-pressing steps.

In another aspect of the invention 5-amino-2,3-dihydro-1,4-phthalazinedione or one of its pharmaceutically acceptable salts or a pharmaceutical composition according to the disclosure are provided in hard gelatin capsules. They are fabricated by producing a powder mixture as described before and filling it into shaped gelatin covers. Glidants and lubricants such as highly dispersed silica, talcum, magnesium stearate, calcium stearate or polyethylene glycol can be added to the powder mixture as solids. A disintegrant or solubilizer such as agar agar, calcium carbonate or sodium carbonate can be added likewise for improving the availability of the medication after intake of the capsule. Additionally, suitable binding agents and/or colorants can be added to the mixture, if desirable or necessary.

In another aspect of the invention 5-amino-2,3-dihydro-1,4-phthalazinedione or one of its pharmaceutically acceptable salts or a pharmaceutical composition according to the disclosure are included in soft gelatin capsules (SGC). SGCs are dissolved on their passage through the gastrointestinal tract. They consist mainly of gelatin enriched with variable amounts of plasticizers such as glycerol or sorbitan. The release rate depends on the specific formulation of the SGC carrier material. They are also suitable for a sustained release of the active agent. SGCs are particularly useful for the administration of poorly water-soluble active agents.

In another aspect of the invention 5-amino-2,3-dihydro-1,4-phthalazinedione or one of its pharmaceutically acceptable salts or a pharmaceutical composition according to the disclosure are included in chewable tablets or hard caramels. Herein the substance is integrated into the matrix of the tablets or caramels.

In another aspect of the invention the present application relates to 5-amino-2,3-dihydro-1,4-phthalazinedione or one of its pharmaceutically acceptable salts or a pharmaceutical composition according to the disclosure for use in the prophylaxis or treatment of a thrombotic disorder in a formulation for inhalatory administration.

For an effective inhalatory prophylaxis or treatment of a thrombotic disorder that at one stage of the disease course affects the respiratory organs it is advantageous that 5-amino-2,3-dihydro-1,4-phthalazinedione or one of its pharmaceutically acceptable salts or a pharmaceutical composition according to the disclosure reaches the patient's alveoli. Therefore, the particle size must be sufficiently small to reach the lowest parts of the airways of the pulmonary tissue. The best inhalatory device class for inhalatory application of a pharmaceutically active agent are the so-called mesh nebulizers. In the scope of the present application practically all mesh nebulizers known in the art can be used, from rather simple single-use mesh nebulizers for cough and cold or for fancy purposes to sophisticated high-end mesh nebulizers for clinical or domestic treatment of serious diseases or conditions of the lower airways.

Suitable commercially available mesh nebulizers, jet nebulizers, ultrasonic nebulizers, dry powder inhalers and (pressurized) metered-dose inhalers comprise, without being limiting, PARI eFlow^{®}rapid, PARI LC STAR^{®}, PARI Velox and PARI Velox Junior (PARI GmbH, Starnberg, Germany), Philips Respironics I-neb and Philips InnoSpire Go (Koninklijke Philips N.V., Eindhoven, Netherlands), VENTA-NEB^{®}-ir, OPTI-NEB^{®}, M-neb^{®} dose⁺ mesh nebulizer inhalation MN-300/8, M-Neb Flow+ and M-neb^{®} mesh nebulizer MN-300/X (NEBU-TEC, Eisenfeld, Germany), Hcmed Deepro HCM-86C and HCM860 (HCmed Innovations Co., Ltd, Taipei, Taiwan), OMRON MicroAir U22 and U100 (OMRON, Kyoto, Japan), Aerogen^{®} Solo, Aerogen^{®} Ultra and Aerogen^{®} PRO (Aerogen, Galway, Ireland), KTMED NePlus NE-SM1 (KTMED Inc., Seoul, South Korea), Vectura Bayer Breelib^{™} (Bayer AG, Leverkusen, Germany), Vectura Fox, MPV Truma and MicroDrop^{®} Smarty (MPV MEDICAL GmbH, Kirchheim, Germany), MOBI MESH (APEX Medical, New Taipei City, Taiwan), B.Well WN-114, TH-134 and TH-135 (B.Well Swiss AG, Widnau, Switzerland), Babybelle Asia BBU01 (Babybelle Asia Ltd., Hongkong), CA-MI Kiwi and others (CA-MI sri, Langhirano, Italy), Diagnosis PRO MESH (Diagnosis S.A., Bia 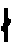 ystok, Poland), DIGI O₂ (DigiO₂ International Co., Ltd., New Taipei City, Taiwan), feellife AIR PLUS, AEROCENTRE+, AIR 360+, AIR GARDEN, AIRICU, AIR MASK, AIRGEL BOY, AIR ANGEL, AIRGEL GIRL and AIR PRO 4 (Feellife Health Inc., Shenzhen, China), Hannox MA-02 (Hannox International Corp., Taipei, Taiwan), Health and Life HL100 and HL100A (HEALTH & LIFE Co., Ltd., New Taipei City, Taiwan), Honsun NB-810B (Honsun Co., Ltd., Nantong, China), K-jump^{®} KN-9100 (K-jump Health Co., Ltd., New Taipei City, Taiwan), microlife NEB-800 (Microlife AG, Widnau, Switzerland), OK Biotech Docspray (OK Biotech Co., Ltd., Hsinchu City, Taiwan), Prodigy Mini-Mist^{®} (Prodigy Diabetes Care, LLC, Charlotte, USA), Quatek NM211, NE203, NE320 and NE403 (Big Eagle Holding Ltd., Taipei, Taiwan), Simzo NBM-1 and NBM-2 (Simzo Electronic Technology Ltd., Dongguan, China), Mexus^{®} BBU01 and BBU02 (Tai Yu International Manufactory Ltd., Dongguan, China), TaiDoc TD-7001 (TaiDoc Technology Co., New Taipei City, Taiwan), Vibralung^{®} and HIFLO Miniheart Circulaire II (Westmed Medical Group, Purchase, USA), KEJIAN (Xuzhou Kejian Hi-Tech Co., Ltd., Xuzhou, China), YM-252, P&S-T45 and P&S-360 (TEKCELEO, Valbonne, France), Maxwell YS-31 (Maxwell India, Jaipur, India), Kernmed^{®} JLN-MB001 (Kernmed, Durmersheim, Germany).

Preferred are mesh nebulizers with a piezoelectric activation of the nebulization process, respectively vibrating mesh nebulizers.

Thus, in another aspect of the invention the present application relates to 5-amino-2,3-dihydro-1,4-phthalazinedione or one of its pharmaceutically acceptable salts or a pharmaceutical composition according to the disclosure for use in the prophylaxis or treatment of a thrombotic disorder in a formulation for inhalatory administration, wherein the inhalatory administration is carried out by means of a vibrating mesh nebulizer.

Mesh nebulizers can be classified into two groups according to patient interaction: Continuous mode devices and trigger-activated devices. In continuous mode mesh nebulizers, the nebulized aerosol is continuously released into the mouthpiece and the patient has to inhale the provided aerosol. In trigger-activated devices a defined amount of aerosol is released only upon an active and deep inspiratory breath. This way a far larger amount of active agent-containing aerosol is inhaled and reaches the lowest airways than with continuous mode devices. The latter lose a large amount of active agent-containing aerosol either to the surrounding or on the passage of the upper airways, as the aerosol release is not coupled to the respiratory cycle.

Therefore, trigger-activated mesh nebulizers are preferred, in particular vibrating mesh nebulizers.

Particularly preferred are trigger-activated mesh nebulizers with a piezoelectric activation of the nebulization process.

Preferred are the mesh nebulizer models PARI eFlow^{®}rapid, Philips Respironics I-neb, Philips InnoSpire Go, M-neb^{®} dose⁺ mesh nebulizer inhalation MN-300/8, Hcmed Deepro HCM-86C and HCM860, OMRON MicroAir U100, Aerogen^{®} Solo, KTMED NePlus NE-SM1, Vectura Fox, Vectura Bayer Breelib^{™}.

The most preferred vibrating mesh nebulizer models are high-end models such as PARI eFlow^{®}rapid, PARI Velox, Philips Respironics I-neb, M-neb^{®} dose⁺ mesh nebulizer inhalation MN-300/8, Aerogen^{®} Solo, Vectura Fox, Vectura Bayer Breelib^{™}.

The mean droplet size is usually characterized as MMAD (median mass aerodynamic diameter). The individual droplet size is referred to as MAD (mass aerodynamic diameter). This value indicates the diameter of the nebulized particles (droplets) at which 50% are smaller or larger, respectively. Particles with a MMAD > 10 µm normally do not reach the lower airways, they often get stuck in the throat. Particles with a MMAD > 5 µm and < 10 µm usually reach the bronchi but not the alveoli. Particles between 100 nm and 1 µm MMAD do not deposit in the alveoli and are exhaled immediately. Therefore, the optimal range is between 1 µm and 5 µm MMAD. Recent publications even favor a narrower range between 3.0 µm and 4.0 µm (cf. Amirav et al. (2010) J Allergy Clin Immunol 25: 1206-1211; Haidl et al. (2012) Pneumologie 66: 356-360).

A further commonly accepted quality parameter is the percentage of the particles in the generated aerosol with a diameter in the range of 1 µm to 5 µm (FPM; fine particle mass). FPM is a measure for the particle distribution. It is calculated by subtracting the percentage of the particles in the generated aerosol with a diameter in the range < 1 µm from the overall percentage of the particles in the generated aerosol with a diameter in the range < 5 µm (FPF; fine particle fraction).

In another aspect of the invention the present application refers also to a method for producing an aerosol according to the disclosure for the prophylaxis or treatment of a thrombotic disorder, comprising the following steps:
a) filling 0.1 ml to 5 ml of an aqueous solution containing 5-amino-2,3-dihydro-1,4-phthalazinedione or one of its pharmaceutically acceptable salts or a pharmaceutical composition according to the disclosure and optionally at least one pharmaceutically acceptable excipient into the nebulization chamber of a mesh nebulizer,
b) starting vibration of the mesh of the mesh nebulizer at a frequency of 80 kHz to 200 kHz, and
c) discharging the generated aerosol at the side of the mesh of the mesh nebulizer opposite to the nebulization chamber.

The vibration frequency of vibrating mesh nebulizers is normally in the range of 80 kHz to 200 kHz, preferred 90 kHz to 180 kHz, more preferred 100 kHz to 160 kHz, most preferred 105 kHz to 130 kHz (cf. Chen, The Aerosol Society: DDL2019**;** Gardenshire et al. (2017) A Guide to Aerosol Delivery Devices for Respiratory Therapists, 4th ed.).

Thus, the beforementioned method is also disclosed with said vibration frequency ranges.

The method according to the disclosure is thus characterized in that at least 80 % in weight, preferred at least 85 % in weight, most preferred at least 90 % in weight of 5-amino-2,3-dihydro-1,4-phthalazinedione or one of its pharmaceutically acceptable salts or a pharmaceutical composition according to the disclosure contained in said aqueous solution are nebulized in the generated aerosol.

The method of the invention is particularly effective in nebulizing a high percentage of the pharmaceutically active agent(s) from the provided aqueous solution during a short time. This is an important feature for patient compliance. A considerable percentage of the patient population finds the inhalatory process to be uncomfortable, weary and physically demanding. On the other hand, the patient's active cooperation is essential for an effective and targeted inhalatory application. Therefore, it is desirable that a therapeutically sufficient amount is applied during a period of time as short as possible. Surprisingly, it showed that during a three minutes' time span 95 % of the substance provided in the aqueous solution could be nebulized. This is an ideal time span for a high patient compliance.

Therefore, the method according to the disclosure is thus characterized in that at least 80 % of the generated aerosol are produced during three minutes after starting nebulization in the mesh nebulizer, preferred at least 85 % and most preferred at least 90 %.

While pharmaceutically active agents are usually provided in a single dosage container for every nebulization procedure the nebulizer and/or the mouthpiece can be used over a certain period of time and have to be replaced at certain intervals. A cleaning of the nebulizer and the mouthpiece is recommended by default after each nebulization. But herein patient compliance cannot be reasonably taken for granted. But even after a meticulous cleaning there are always some deposits of the aerosol in the nebulization chamber, the outlet and/or the mouthpiece. As the aerosol is produced from an aqueous solution these depositions bear the risk of producing a bioburden of bacteria that might contaminate the inhaled aerosol. Deposits may also plug holes in the mesh membrane of the mesh nebulizer. In general, the nebulizer and/or the mouthpiece should be exchanged every one or two weeks. Therefore, it is convenient to offer the medication and the nebulizer as a combined product.

Thus, in another aspect of the invention the present application refers also to a kit comprising a mesh nebulizer and a pharmaceutically acceptable container with an aqueous solution containing an effective amount of 5-amino-2,3-dihydro-1,4-phthalazinedione or one of its pharmaceutically acceptable salts or a pharmaceutical composition according to the disclosure for the prophylaxis or treatment of a thrombotic disorder and optionally at least one pharmaceutically acceptable excipient.

In an alternative kit 5-amino-2,3-dihydro-1,4-phthalazinedione or one of its pharmaceutically acceptable salts or a pharmaceutical composition according to the disclosure is not provided in form of an aqueous solution but in two separated containers, one for a solid form for the active agent and the other for an aqueous solution. The final aqueous solution is freshly prepared by solving the active agent in the final solution. Thereupon the final aqueous solution is filled into the nebulization chamber of the mesh nebulizer. These two containers can be completely separated containers e.g., two vials, or e.g., a dual-chamber vial. For solving the active agent e.g., a membrane between the two chambers is perforated to allow for mixing of the content of both chambers.

Thus, the present application discloses also a kit, comprising a mesh nebulizer, a first pharmaceutically acceptable container with water for injection or physiological saline solution and a second pharmaceutically acceptable container with an effective dosage of a solid form of 5-amino-2,3-dihydro-1,4-phthalazinedione or one of its pharmaceutically acceptable salts or a pharmaceutical composition according to the disclosure for the prophylaxis or treatment of a thrombotic disorder, wherein optionally at least one pharmaceutically acceptable excipient is contained in the first pharmaceutically acceptable container and/or the second pharmaceutically acceptable container.

The aerosol generated by the method according to the disclosure is administered, respectively self-administered by means of a mouthpiece. Optionally, such a mouthpiece can be additionally included in the beforementioned kits.

A common way to transfer the provided aqueous solution or final aqueous solution into the nebulization chamber of the mesh nebulizer by means of a syringe equipped with an injection needle. First, the aqueous solution is drawn up into the syringe and then injected into the nebulization chamber. Optionally, such a syringe and/or injection needle can be additionally included in the beforementioned kits. Without being limiting, typical syringes made of polyethylene, polypropylene or cyclic olefin co-polymers can be used, and a typical gauge for a stainless-steel injection needle would be in the range of 14 to 27.

In yet another aspect of the invention 5-amino-2,3-dihydro-1,4-phthalazinedione or one of its pharmaceutically acceptable salts or a pharmaceutical composition according to the disclosure for use in the prophylaxis or treatment of a thrombotic disorder, wherein said substance, composition or combination is applied in form of liposomes, micelles, multilamellar vesicles or a cyclodextrin complex.

In yet another aspect of the invention, the present application discloses 5-amino-2,3-dihydro-1,4-phthalazinedione or one of its pharmaceutically acceptable salts or a composition according to the invention for use in the prophylaxis or treatment of a thrombotic disorder, wherein a previous treatment with at least one other pharmaceutically active agent was refractory.

In yet another aspect of the invention, the present application discloses 5-amino-2,3-dihydro-1,4-phthalazinedione or one of its pharmaceutically acceptable salts or a composition according to the invention for use in the prophylaxis or treatment of a thrombotic disorder, wherein said substance or said pharmaceutical composition is formulated as a suppository.

To produce a dosage form of 5-amino-2,3-dihydro-1,4-phthalazinedione as a suppository, waxes with a low melting point as well as a mixture of fatty acid glycerides such as cocoa butter are first melted, then 5-amino-2,3-dihydro-1,4-phthalazinedione is homogenously dispersed under stirring or other mixing methods. The molten homogeneous mixture is then transferred to suitable molds and cooled down until solidification.

In yet another aspect of the invention the present application relates to 5-amino-2,3-dihydro-1,4-phthalazinedione or one of its pharmaceutically acceptable salts or a composition according to the invention for use in the prophylaxis or treatment of a thrombotic disorder in a liquid dosage form.

The present application also discloses the parenteral administration of 5-amino-2,3-dihydro-1,4-phthalazinedione or one of its pharmaceutically acceptable salts or a composition according to the invention for use in the prophylaxis or treatment of a thrombotic disorder in the form of intravenous injection, intraarterial injection or intraperitoneal injection.

These liquid dosage forms comprise solutions, suspensions and emulsions. Examples are water and water/propylene glycol solutions for parenteral injections, or the addition of a sweetener or opacifier for oral solutions, suspensions and emulsions.

These liquid dosage forms can be stored in vials, IV bags, ampoules, cartridges, and prefilled syringes. Suitable excipients include solubilizers, stabilizers, buffers, tonicity modifiers, bulking agents, viscosity enhancers/reducers, surfactants, chelating agents, and adjuvants.

A preferred dosage form according to the invention are retard formulations i.e., formulations with a delayed release of the at least one active agent. They are also known as sustained release (SR), extended release *(ER, XR)* or controlled/continuous release (CR) forms. Suitable formulations and carriers are known to a person skilled in the art (Kleinsorge (1995) Retardformulierungen in der medikamentösen Therapie. Leipzig, Barth 8th ed.). Most commonly, the active agent is embedded in a matrix of insoluble substances like acrylics or chitin. Hence, the active agent must find its way out through orifices in the matrix. In some formulations, there is a laser-drilled hole on one side and opposite to it a porous membrane. The gastric fluid attacks this porous membrane, flows in and pushes the active agent through the drilled hole on the opposite side. In other formulations, the active agent dissolves inside the matrix swelling thereupon and forming a gel. Then the active agent is released through the pores of the gel. Other examples include specifically coated tablets resistant to gastric fluid, retard capsules containing retard pellets of the active agent that are going to be released after the dissolution of the capsule casing, multiple unit pellet systems (MUPS), oral osmotic systems, resonates, coacervation and micro-encapsulation. With the use of such a retard formulation the release site of a drug and its pharmacokinetics can be controlled. For example, it is often desirable that a dosage form of an active agent is not dissolved before reaching a certain point of the intestines. As the pH changes along the way through the intestines, the dissolution process may be engineered to be pH dependent. In therapeutic applications in which the absorption of an active agent through the intestinal mucosa shall be facilitated for increasing its bioavailability it may be preferable not to use a salt of an active agent but its neutral form.

Therefore, the present application refers also to 5-amino-2,3-dihydro-1,4-phthalazinedione or one of its pharmaceutically acceptable salts or a composition according to the invention for use in the prophylaxis or treatment of a thrombotic disorder, wherein said substance or said pharmaceutical composition is formulated as a retard drug.

In yet another aspect of the invention 5-amino-2,3-dihydro-1,4-phthalazinedione or one of its pharmaceutically acceptable salts or a composition according to the invention for use in the prophylaxis or treatment of a thrombotic disorder, wherein said substance, composition or combination is formulated as a lyophilizate. A lyophilizate can be reconstituted with water for injection or physiological saline or a water/ethanol solution and then be administered by injection.

Typical application forms for intravenous injections include infusion pumps, hypodermic needles, drip chambers, peripheral cannulas (peripheral venous catheters) and pressure bags.

In general, an aqueous solution or a physiological saline solution is preferred. In case of a poorly soluble pharmaceutical agent according to the disclosure also ethanol or ethanol/water mixtures can be used.

Further suitable liquid dosage forms are drops, gels and hydrogels.

A gel is a colloid in which the solid disperse phase forms a network in combination with that of the fluid continuous phase, resulting in a viscous semirigid sol. Gel properties range from soft and weak to hard and tough. They are defined as a substantially dilute cross-linked system, which exhibits no flow in the steady state. By weight, gels are mostly liquid, yet they behave like solids due to a three-dimensional cross-linked network within the liquid. It is the crosslinking within the fluid that gives a gel its consistency and contributes to the adhesive stick. Gels are a dispersion of molecules of a liquid within a solid medium.

A hydrogel is a network of polymer chains that are hydrophilic, sometimes found as a colloidal gel in which water is the dispersion medium. A three-dimensional solid results from the hydrophilic polymer chains being held together by cross-links. Because of the inherent cross-links, the structural integrity of the hydrogel network does not dissolve from the high concentration of water. Hydrogels are highly absorbent (they can contain over 90% water) natural or synthetic polymeric networks. Hydrogels also possess a degree of flexibility very similar to natural tissue, due to their significant water content. In medicine, hydrogels can encapsulate chemical systems which upon stimulation by external factors such as a change of pH may cause specific pharmaceutically active agent(s) to be liberated to the environment, in most cases by a gel-sol transition to the liquid state.

Suitable gel formers can be selected from the group comprising, but not limited to, agar, algin, alginic acid, bentonite, carbomer, carrageenan, hectorite, hydroxyethyl cellulose, hydroxypropyl cellulose, polyvinyl alcohol, polyvinyl pyrrolidone, sodium carbomer.

In yet another aspect of the invention the present application relates to 5-amino-2,3-dihydro-1,4-phthalazinedione or one of its pharmaceutically acceptable salts or a composition according to the invention for use in the prophylaxis or treatment of a thrombotic disorder in a formulation for sublingual tablets.

Sublingual drug delivery can be an alternative when compared to oral drug delivery as sublingually administered dosage forms bypass hepatic metabolism. A rapid onset of pharmacological effect is often desired for some drugs, especially those used in the treatment of acute disorders. Sublingual tablets disintegrate rapidly, and the small amount of saliva present is usually sufficient for achieving disintegration of the dosage form coupled with better dissolution and increased bioavailability.

The drug must be lipophilic enough to be able to partition through the lipid bilayer, but not so lipophilic such that once it is in the lipid bilayer, it will not partition out again. According to the diffusive model of absorption, the flux across the lipid bilayer is directly proportional to the concentration gradient. Therefore, lower salivary solubility results in lower absorption rates and vice versa. In general, a drug which has been formulated for sublingual should ideally have a molecular weight of less than 500 to facilitate its diffusion. The oral cavity has a narrow pH range which lies between 5.0 to 7.0. The inclusion of a suitable buffer during the formulation of an ionizable drug makes it possible to control the pH of aqueous saliva.

In order to avoid a possibly unpleasant taste or smell of the drug taste masking is needed. Sweeteners, flavors, and other taste-masking agents are essential components. Sugar-based excipients quickly dissolve in saliva and produce endothermic heat of dissolution. They create a pleasant feeling in the mouth and are most suitable for sublingual tablets along with other flavors.

Typical techniques for manufacturing sublingual tablets include direct compression, compression molding, freeze drying and hot melt extrusion (Khan et al. (2017) J Pharmaceut Res 16: 257-267).

When swallowing is avoided, an administration of a pharmaceutically active agent by means of a sublingual tablet can also reach the pharynx/throat topically. Absorption of the pharmaceutically active agent occurs to a good part via the pharyngeal mucosa.

In yet another embodiment of the invention a pharmaceutical composition comprising 5-amino-2,3-dihydro-1,4-phthalazinedione or one of its pharmaceutically acceptable salts for use in the prophylaxis or treatment of a thrombotic disorder is provided as a topical application form, such as creams, emulsions, lotions, gels, hydrogels, pastes, powders, ointments, liniment, films, liposomes, dermal patches, transdermal patches, transdermal sprays or suspensions.

In a further aspect of the invention a method of prophylaxis or treatment of a thrombotic disorder, is disclosed, in which an effective dose of 5-amino-2,3-dihydro-1,4-phthalazinedione or one of its pharmaceutically acceptable salts or a pharmaceutical composition according to the disclosure is administered to a patient in need thereof.

### EXAMPLES

### Example 1:

In a Phase Ila clinical study the efficacy of 300 mg 5-amino-2,3-dihydro-1,4-phthalazinedione sodium salt Form I plus SoC (Standard of Care) versus placebo plus SoC was evaluated in hospitalized patients with moderate to severe COVID-19 (EudraCT-No.: 2021-000344-21, IND 153604). The study was randomized, double-blind, and placebo-controlled, with enrollment at 20 IEC (independent ethics committee)-approved sites across six countries (Bulgaria, France, Hungary, Italy Romania, Spain); a list of all participating sites is published on clinicaltrials.gov. (NCT04932941). This study was partially funded by the COVID-19 Horizon Europe work programme (Project No.: 101046182). The funder of the study had no role in study design, data collection, data analysis, data interpretation, or writing of the report.

Patients aged 18 years or older with a positive SARS-CoV-2 test and symptoms suggestive of moderate or severe systemic illness were eligible for enrollment, while those with asymptomatic or mild COVID-19, or critical COVID-19 on day 1, were excluded. Further details on the in- and exclusion criteria are available on cliniclatrials.gov NCT04932941.

Patients were randomized 2:1 to receive either 300 mg 5-amino-2,3-dihydro-1,4-phthalazinedione sodium salt Form I BID (twice daily) plus SoC or placebo BID plus SoC using an interactive web response system (IWRS) based on a randomization scheme developed by an unblinded, independent statistician. Stratification factors included baseline COVID-19 severity and age-class (aged ≤ 65 years versus > 65 years).

Hard gelatine capsules of 50 mg 5-amino-2,3-dihydro-1,4-phthalazinedione sodium salt Form I and placebo capsules were supplied in identical blister packaging and were color- and size-matched, with blinded labelling.

Sample size was determined using the Chi-square test with type I error alpha=10% two-sided for this proof-of-concept study and 2:1 randomization ratio, 114 patients (76 in Arm A and 38 in Arm B) were required to achieve a statistical power of 83%. Considering about 5% early study terminations, the necessary sample size for randomisation resulted in 120 patients in total (80 in Arm A and 40 in Arm B). As the early study termination rate was higher than the estimated 5%, the number of randomised patients was increased during the study to ensure 114 evaluable patients at day 14. Sample size was estimated using nQuery 8, version 8.6.1.0.

The Standard of Care treatments used in this study were generally balanced between the two groups, with oxygen being the most common treatment used in over 99% of patients overall. Other common treatments included dexamethasone (61.4% of patients overall); sodium chloride (54.5% of patients overall); paracetamol (38.6% of patients overall); and methylprednisolone, ceftriaxone, pantoprazole, and ascorbic acid (all 35.6% of patients overall). The use of antivirals for systemic use was also balanced between both groups with 52 patients (39.4% of patients overall) receiving antivirals with remdesivir being the most commonly administered (28.8% of patients).

Patients were treated for 28 days. The majority of patients showed disease resolution by Day 14 (69/82 in the verum group, 31/43 in the placebo group). These patients were discharged from the hospital before and received medication (verum or placebo) for continuing self-treatment for the remaining time at home. After 60 days there was a follow-up visit.

Besides the parameters for the clinical outcome of this study an exposure-response analysis was performed to establish a pharmacokinetic (PK) model of 5-amino-2,3-dihydro-1,4-phthalazinedione sodium salt Form I. To this aim, the biomarker D-Dimer was determined amongst others.

The D-dimer level is an important prognostic marker for COVID-19 severity. An elevated D-dimer level is associated with thromboembolic complications. For monitoring the development of this biomarker throughout the study blood samples were taken at Days 1, 7, 14, 21, 28 and 60. The D-dimer concentration was determined (PPD Central Lab, Zaventem, Belgium) from the plasma by means of monoclonal antibodies quantified in an ELISA (cf. Olson (2015) Adv Clin Chem 69: 1 - 46; Johnson et al. (2019) Am J Hematol 94: 833-839). Results are presented in Table 1.

**Table 1:**

| **treatment day** | **5-amino-2,3-dihydro-1,4-phthalazinedione sodium salt Form I + SoC** | **n** | **placebo + SoC** | **n** |
|---|---|---|---|---|
| 1 | 235.5 | 84 | 259.0 | 41 |
| 7 | 205.0 | 80 | 283.0 | 39 |
| 14 | 171.0 | 75 | 197.5 | 42 |
| 21 | 208.0 | 66 | 242.5 | 30 |
| 28 | 172.0 | 74 | 266.5 | 34 |
| 60 | 138.0 | 67 | 139.5 | 32 |

D-dimer concentrations vary considerably among individuals, with relevant outliers in both directions. For rendering the results more comparable, the median of the respective group is indicated in ng/mL DDU (D-dimer units).

The group that received 5-amino-2,3-dihydro-1,4-phthalazinedione sodium salt + SoC showed significantly lower values of D-dimer throughout the course of the study, in comparison to the group that received placebo + SoC. An unsmooth course of the curves may be due that blood samples could not be taken at all time points from all patients. Therefore, composition and size of the respective groups vary slightly. Further, patients from both groups who deceased during the time of the study contributed usually elevated D-dimer values only when they were still alive. These patients deceased during the first 14 days. After discharge from hospital the patients did not receive anymore SoC. This could explain the temporary rebound in both groups. After 60 days all surviving patients were healed and the D-dimer values had renormalized, and no difference between the groups could be detected.

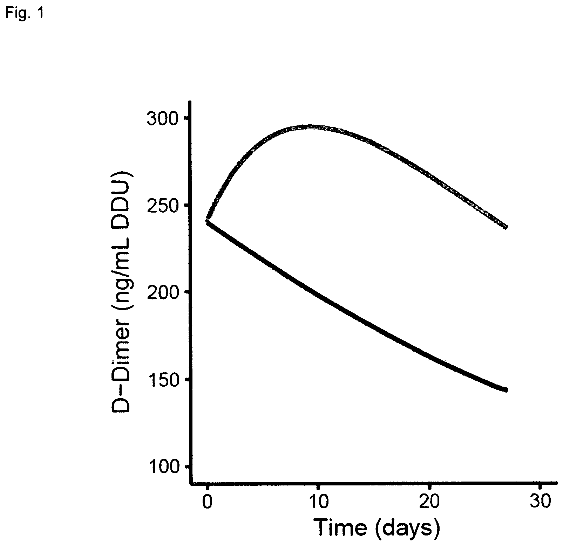

In a post-hoc analysis, the effect of received 5-amino-2,3-dihydro-1,4-phthalazinedione sodium salt + SoC on the biomarker D-dimer was modelled (Saarmetrics GmbH, University of the Saarland, Saarbrücken, Germany). It revealed a significant effect of received 5-amino-2,3-dihydro-1,4-phthalazinedione sodium salt + SoC resulting in a significant decrease of D-dimer levels compared to placebo (p<0.001) (Fig. 1).

It can be learnt from Fig. 1 that 5-amino-2,3-dihydro-1,4-phthalazinedione sodium salt reduces D-dimer in the increasing phase of the curve of the placebo group as well as in the recovery phase of the placebo group. This can be interpreted that 5-amino-2,3-dihydro-1,4-phthalazinedione sodium salt has a prophylactic effect (less D-dimer is formed, therefore there is a less risk of a thrombotic event) as well as a therapeutic effect (formed fibrin thrombi or respective coils are more rapidly degraded and removed).

### Example 2: Effects of 5-amino-2,3-dihydro-1,4-phthalazinedione sodium salt in an in vitro cell system

Different concentrations of 5-amino-2,3-dihydro-1,4-phthalazinedione sodium salt were tested in a cell system whether there is an effect on thrombomodulin release, in comparison to control. Coronary artery smooth muscle cells pooled from human donors (n = 3) were commercially acquired and cultured according to the recommendations of the manufacturer. They were stimulated with IL-1β, TNF-α and IFN-γ to emulate conditions of vascular inflammation. The B cells were cultured in 96 well-plates until confluence. Details are described in Kunkel et al. (2004) FASEB J 18: 1279-1281). 5-amino-2,3-dihydro-1,4-phthalazinedione sodium salt Form I was provided by Applicant. Stock solutions of 5-amino-2,3-dihydro-1,4-phthalazinedione sodium salt were prepared in PBS (phosphate-buffered saline) and thawed before the experiment. The substance was added at the indicated concentration 1 hour before stimulation and remained in culture for 48 hours. Each plate contained positive and negative controls (non-stimulated cells) as well as vehicle controls (buffer solution). Direct ELISA (enzyme-linked immunosorbent assay) was used to measure thrombomodulin levels. Soluble factors from supernatants were quantified using capture ELISA.

Overt cytotoxic effects of 5-amino-2,3-dihydro-1,4-phthalazinedione sodium salt were monitored by SRB (sulforhodamine B) assay for 24 hours. During this time no cytotoxic effects were observed for any of the substances or combinations (data not shown).

The following concentrations and combinations were of 5-amino-2,3-dihydro-1,4-phthalazinedione sodium salt were tested: $62.5 μM - 250 μM - 1 mM - 4 mM$

Measurements were carried out in triplicates which were then averaged and divided by the average of vehicle control samples. Vehicle control samples were taken as 100%. The following results were achieved:

**Table 2:**

| | | | | |
|---|---|---|---|---|
| control | 62.5 µM | 250 µM | 1 mM | 4 mM |
| 100 % | 127.4 % | 115.1 % | 159.6 % | 255.9 % |

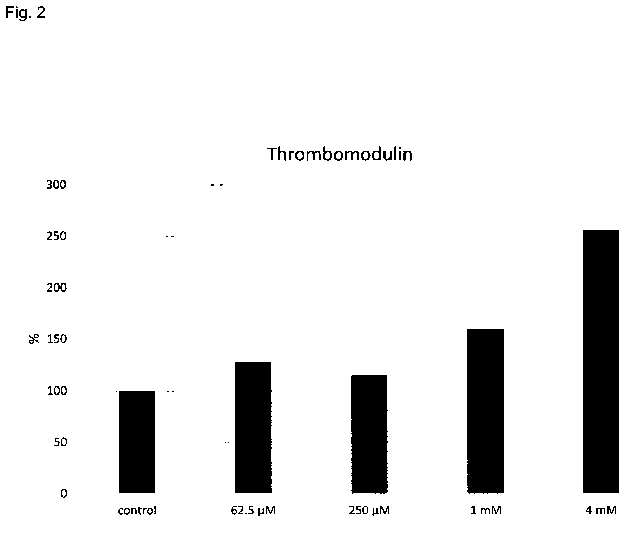

The results are depicted in Fig. 2.

These results show that the prophylactic addition of an effective concentration of 5-amino-2,3-dihydro-1,4-phthalazinedione sodium salt is able to significantly increase the release of thrombomodulin from cardiovascular smooth muscle cells in a dose-dependent manner. Hence, the results suggest that the addition of 5-amino-2,3-dihydro-1,4-phthalazinedione sodium salt before stimulation is able to prevent thrombus formation in a vessel under pro-inflammatory conditions. Therefore, it is reasonable to assume that the use of 5-amino-2,3-dihydro-1,4-phthalazinedione sodium salt can be prophylactic against the formation of a thrombus, respectively able to prevent the manifestation of a thrombotic disorder.

### FIGURES

- Fig. 1: shows a modelled course of D-dimer concentrations in the plasma (ng/mL DDU) over the course of the clinical study.
black line: 5-amino-2,3-dihydro-1,4-phthalazinedione sodium salt + SoC
grey Line: Placebo + SoC
- Fig. 2: shows the respective change in % of thrombomodulin towards control in the supernatant after adding different concentrations of 5-amino-2,3-dihydro-1,4-phthalazinedione sodium salt. Values were normalized to 100% (control).

## Claims

1. 5-amino-2,3-dihydro-1,4-phthalazinedione or one of its pharmaceutically acceptable salts, hydrates or solvates for use in the prophylaxis and treatment of a thrombotic disorder.

2. 5-amino-2,3-dihydro-1,4-phthalazinedione or one of its pharmaceutically acceptable salts, hydrates or solvates for use according to claim 1, wherein said pharmaceutically acceptable salt is 5-amino-2,3-dihydro-1,4-phthalazinedione sodium salt.

3. 5-amino-2,3-dihydro-1,4-phthalazinedione or one of its pharmaceutically acceptable salts, hydrates or solvates for use according to claim 2, wherein 5-amino-2,3-dihydro-1,4-phthalazinedione sodium salt is provided as one of crystalline anhydrate polymorph forms I, II or III **characterized by** crystallography values determined by means of X-ray powder diagrams:
d values: 13.5; 6.9; 5.2; 4.6; 3.9; 3.5; 3.4; 3.3; 3.1; 3.0 and/or 2-theta values: 6.5; 12.7; 16.9; 19.3; 22.8; 25.8; 26.6; 27.2; 28.7; 30.3 for Form I,
d values: 12.9; 7.9; 7.1; 6.5; 5.3; 4.0; 3.7; 3.6; 3.3; 3.2 and/or 2-theta values: 6.8; 11.2; 12.5; 13.7; 16.7; 22.4; 24.3; 24.9; 27.2; 27.8 for Form II, and
d values: 13.131; 7.987; 7.186; 6.566; 6.512; 5.372; 3.994; 3.662; 3.406; 3.288; 3.283; 3.222; 3.215; 3.127; 2.889 and/or
2-theta values: 6.73; 11.07; 12.31; 13.48; 13.59; 16.49; 22.24; 24.29; 26.14; 27.10; 27.14; 27.67; 27.72; 28.52; 30.93 for Form III.

4. 5-amino-2,3-dihydro-1,4-phthalazinedione or one of its pharmaceutically acceptable salts, hydrates or solvates for use according to any one of claims 2 or 3, wherein the daily dosage for a patient in need thereof is in the range of 100 mg to 1000 mg.

5. 5-amino-2,3-dihydro-1,4-phthalazinedione or one of its pharmaceutically acceptable salts, hydrates or solvates for use according to any one of claims 1 to 4, wherein the thrombotic disorder is caused by a viral infection.

6. 5-amino-2,3-dihydro-1,4-phthalazinedione or one of its pharmaceutically acceptable salts, hydrates or solvates for use according to claim 5, wherein the thrombotic disorder is caused by a SARS-CoV-2 infection.

7. 5-amino-2,3-dihydro-1,4-phthalazinedione or one of its pharmaceutically acceptable salts for use according to any one of claims 1 to 4, wherein the thrombotic disorder is selected from a group comprising venous thrombosis, deep vein thrombosis, Paget-Schroetter disease, Budd-Chiari syndrome, portal vein thrombosis, renal vein thrombosis, cerebral venous sinus thrombosis, jugular vein thrombosis, cavernous sinus thrombosis, thrombophlebitis, perinatal thrombosis, phlegmasia alba dolens, arterial thrombosis, peripheral artery disease, acute limb ischemia, hepatic artery thrombosis, disseminated intravascular coagulation, coagulation dysfunction in septic disorders and thrombotic microangiopathy.

8. Pharmaceutical composition containing 5-amino-2,3-dihydro-1,4-phthalazinedione or one of its pharmaceutically acceptable salts, hydrates or solvates and at least one pharmaceutically acceptable excipient for use in the in the prophylaxis or treatment of a thrombotic disorder.

9. Pharmaceutical composition for use according to claim 8, wherein said pharmaceutical composition is suitable for inhalatory, oral, parenteral, intraperitoneal, intravenous, intraarterial, intramuscular, topical, transdermal, subcutaneous, intradermal, sublingual, conjunctival, intravaginal, rectal, intrathecal, pharyngeal or nasal administration or by intubation.

10. Pharmaceutical composition for use according to any one of claims 8 or 9, wherein said at least one pharmaceutically acceptable excipient is selected from a group comprising carriers, binding agents, colorants, buffers, preservatives, antioxidants, coatings, sweeteners, thickening agents, pH-regulators, acidity regulators, acidifiers, solvents, isotonizing agents, disintegrants, glidants, lubricants, emulsifiers, solubilizing agents, stabilizers, diluents, anti-caking agents, sorbents, permeation enhancers, foaming agents, anti-foaming agents, opacifiers, fatliquors, consistency enhancers, hydrotropes, aromatic and flavoring substances.

11. 5-amino-2,3-dihydro-1,4-phthalazinedione or one of its pharmaceutically acceptable salts, hydrates or solvates for use according to claim 1 or a pharmaceutical composition for use according to claim 8, wherein the oral dosage form of said substance or said pharmaceutical composition is in the form of tablets, sublingual tablets, soft gelatin capsules, hard gelatin capsules, sugar-coated tablets or pills; powders or granulates; juices, syrups, drops, teas, solutions or suspensions in aqueous or non-aqueous liquids; edible foams or mousses; lozenges; or in oil-in-water or water-in-oil in emulsions.

12. 5-amino-2,3-dihydro-1,4-phthalazinedione or one of its pharmaceutically acceptable salts, hydrates or solvates for use according to claim 1 or a pharmaceutical composition for use according to claim 8, wherein said substance or said pharmaceutical composition is applied by inhalation by using a vibrant mesh nebulizer, metered dose-inhaler, jet nebulizer or dry-powder inhaler.

13. 5-amino-2,3-dihydro-1,4-phthalazinedione or one of its pharmaceutically acceptable salts, hydrates or solvates for use according to claim 1 or a pharmaceutical composition for use according to claim 8, wherein said substance or said pharmaceutical composition is formulated as a retard drug.

14. 5-amino-2,3-dihydro-1,4-phthalazinedione or one of its pharmaceutically acceptable salts, hydrates or solvates for use according to claim 1 or a pharmaceutical composition for use according to claim 8, wherein said substance or said pharmaceutical composition is applied in form of liposomes, micelles, multilamellar vesicles or a cyclodextrin complex.

15. 5-amino-2,3-dihydro-1,4-phthalazinedione or one of its pharmaceutically acceptable salts, hydrates or solvates for use according to claim 1 or a pharmaceutical composition for use according to claim 8, wherein said substance or said pharmaceutical composition is applied topically in the form of creams, emulsions, lotions, gels, hydrogels, pastes, powders, ointments, liniment, films, liposomes, dermal patches, transdermal patches, transdermal sprays or suspensions.

## Patentansprüche

1. 5-Amino-2,3-dihydro-1,4-phthalazindion oder eines seiner pharmazeutisch verträglichen Salze, Hydrate oder Solvate zur Verwendung bei der Prophylaxe und Behandlung einer thrombotischen Erkrankung.

2. 5-Amino-2,3-dihydro-1,4-phthalazindion oder eines seiner pharmazeutisch verträglichen Salze, Hydrate oder Solvate zur Verwendung gemäß Anspruch 1, wobei das pharmazeutisch verträgliche Salz 5-Amino-2,3-dihydro-1,4-phthalazindion Natriumsalz ist.

3. 5-Amino-2,3-dihydro-1,4-phthalazindion oder eines seiner pharmazeutisch verträglichen Salze, Hydrate oder Solvate zur Verwendung gemäß Anspruch 2, wobei das Natriumsalz von 5-Amino-2,3-dihydro-1,4-phthalazindion als eine der kristallinen anhydrischen Polymorphe I, II oder III vorliegt, **gekennzeichnet durch** kristallographische Werte, die mittels Röntgenpulverdiffraktogrammen bestimmt wurden:
d-Werte: 13,5; 6,9; 5,2; 4,6; 3,9; 3,5; 3,4; 3,3; 3,1; 3,0 und/oder
2-Theta-Werte: 6,5; 12,7; 16,9; 19,3; 22,8; 25,8; 26,6; 27,2; 28,7; 30,3 für Form I,
d-Werte: 12,9; 7,9; 7,1; 6,5; 5,3; 4,0; 3,7; 3,6; 3,3; 3,2 und/oder
2-Theta-Werte: 6,8; 11,2; 12,5; 13,7; 16,7; 22,4; 24,3; 24,9; 27,2; 27,8 für Form II sowie
d-Werte: 13,131; 7,987; 7,186; 6,566; 6,512; 5,372; 3,994; 3,662; 3,406; 3,288; 3,283; 3,222; 3,215; 3,127; 2,889 und/oder
2-Theta-Werte: 6,73; 11,07; 12,31; 13,48; 13,59; 16,49; 22,24; 24,29; 26,14; 27,10; 27,14; 27,67; 27,72; 28,52; 30,93 für Form III.

4. 5-Amino-2,3-dihydro-1,4-phthalazindion oder eines seiner pharmazeutisch verträglichen Salze, Hydrate oder Solvate zur Verwendung gemäß einem der Ansprüche 2 oder 3, wobei die Tagesdosis für einen Patienten, der dies benötigt, im Bereich von 100 mg bis 1000 mg liegt.

5. 5-Amino-2,3-dihydro-1,4-phthalazindion oder eines seiner pharmazeutisch verträglichen Salze, Hydrate oder Solvate zur Verwendung gemäß einem der Ansprüche 1 bis 4, wobei die thrombotische Erkrankung durch eine Virusinfektion verursacht wird.

6. 5-Amino-2,3-dihydro-1,4-phthalazindion oder eines seiner pharmazeutisch verträglichen Salze, Hydrate oder Solvate zur Verwendung gemäß Anspruch 5, wobei die thrombotische Erkrankung durch eine SARS-CoV-2-Infektion verursacht wird.

7. 5-Amino-2,3-dihydro-1,4-phthalazindion oder eines seiner pharmazeutisch verträglichen Salze zur Verwendung gemäß einem der Ansprüche 1 bis 4, wobei die thrombotische Erkrankung aus einer Gruppe ausgewählt ist, die venöse Thrombose, tiefe Venenthrombose, Paget-Schroetter-Syndrom, Budd-Chiari-Syndrom, Pfortaderthrombose, Nierenvenenthrombose, zerebrale Sinusthrombose, Jugularvenenthrombose, Kavernensinusthrombose, Thrombophlebitis, perinatale Thrombose, Phlegmasia alba dolens, arterielle Thrombose, periphere arterielle Verschlusskrankheit, akute Extremitätenischämie, Leberarterienthrombose, disseminierte intravaskuläre Gerinnung, Gerinnungsstörung bei septischen Erkrankungen und thrombotische Mikroangiopathie umfasst.

8. Pharmazeutische Zusammensetzung, die 5-Amino-2,3-dihydro-1,4-phthalazindion oder eines seiner pharmazeutisch verträglichen Salze, Hydrate oder Solvate sowie mindestens einen pharmazeutisch verträglichen Hilfsstoff zur Verwendung bei der Prophylaxe oder Behandlung einer thrombotischen Erkrankung enthält.

9. Pharmazeutische Zusammensetzung zur Verwendung gemäß Anspruch 8, wobei die pharmazeutische Zusammensetzung zur inhalativen, oralen, parenteralen, intraperitonealen, intravenösen, intraarteriellen, intramuskulären, topischen, transdermalen, subkutanen, intradermalen, sublingualen, konjunktivalen, intravaginalen, rektalen, intrathekalen, pharyngealen oder nasalen Verabreichung oder zur Verabreichung mittels Intubation geeignet ist.

10. Pharmazeutische Zusammensetzung zur Verwendung gemäß einem der Ansprüche 8 oder 9, wobei der mindestens eine pharmazeutisch verträgliche Hilfsstoff aus einer Gruppe ausgewählt ist, die Trägerstoffe, Bindemittel, Farbstoffe, Puffer, Konservierungsmittel, Antioxidantien, Beschichtungen, Süßstoffe, Verdickungsmittel, pH-Regulatoren, Säureregulatoren, Säuerungsmittel, Lösungsmittel, Isotonisierungsmittel, Sprengmittel, Gleitmittel, Schmiermittel, Emulgatoren, Lösungsvermittler, Stabilisatoren, Verdünnungsmittel, Antibackmittel, Sorptionsmittel, Permeationsverstärker, Schaumbildner, Entschäumer, Trübungsmittel, Fettungsmittel, Konsistenzverstärker, Hydrotrope sowie Aromastoffe und Geschmacksstoffe umfasst.

11. 5-Amino-2,3-dihydro-1,4-phthalazindion oder eines seiner pharmazeutisch verträglichen Salze, Hydrate oder Solvate zur Verwendung gemäß Anspruch 1 oder eine pharmazeutische Zusammensetzung zur Verwendung gemäß Anspruch 8, wobei die orale Darreichungsform der besagten Substanz oder der besagten pharmazeutischen Zusammensetzung in Form von Tabletten, sublingualen Tabletten, Weichgelatinekapseln, Hartgelatinekapseln, zuckerbeschichteten Tabletten oder Pillen, Pulvern oder Granulaten, Säfte, Sirups, Tropfen, Tees, Lösungen oder Suspensionen in wässrigen oder nichtwässrigen Flüssigkeiten, essbaren Schäumen oder Mousses, Lutschtabletten oder in Öl-in-Wasser- oder Wasser-in-Öl-Emulsionen vorliegt.

12. 5-Amino-2,3-dihydro-1,4-phthalazindion oder eines seiner pharmazeutisch verträglichen Salze, Hydrate oder Solvate zur Verwendung gemäß Anspruch 1 oder eine pharmazeutische Zusammensetzung zur Verwendung gemäß Anspruch 8, wobei die besagte Substanz oder die besagte pharmazeutische Zusammensetzung durch Inhalation unter Verwendung eines Vibrant-Mesh-Verneblers, eines Metered-Dose-Inhalers, eines Jet-Verneblers oder eines Trockenpulverinhalators verabreicht wird.

13. 5-Amino-2,3-dihydro-1,4-phthalazindion oder eines seiner pharmazeutisch verträglichen Salze, Hydrate oder Solvate zur Verwendung gemäß Anspruch 1 oder eine pharmazeutische Zusammensetzung zur Verwendung gemäß Anspruch 8, wobei die besagte Substanz oder die besagte pharmazeutische Zusammensetzung als Retard-Präparat formuliert ist.

14. 5-Amino-2,3-dihydro-1,4-phthalazindion oder eines seiner pharmazeutisch verträglichen Salze, Hydrate oder Solvate zur Verwendung gemäß Anspruch 1 oder eine pharmazeutische Zusammensetzung zur Verwendung gemäß Anspruch 8, wobei die besagte Substanz oder die besagte pharmazeutische Zusammensetzung in Form von Liposomen, Mizellen, multilamellaren Vesikeln oder einem Cyclodextrin-Komplex verabreicht wird.

15. 5-Amino-2,3-dihydro-1,4-phthalazindion oder eines seiner pharmazeutisch verträglichen Salze, Hydrate oder Solvate zur Verwendung gemäß Anspruch 1 oder eine pharmazeutische Zusammensetzung zur Verwendung gemäß Anspruch 8, wobei die besagte Substanz oder die besagte pharmazeutische Zusammensetzung topisch in Form von Cremes, Emulsionen, Lotionen, Gelen, Hydrogelen, Pasten, Pulvern, Salben, eines Einreibemittels, von Filmen, Liposomen, Hautpflastern, transdermalen Pflastern, transdermalen Sprays oder Suspensionen angewendet wird.

## Revendications

1. 5-Amino-2,3-dihydro-1,4-phtalazinedione ou l'un de ses sels, hydrates ou solvates pharmaceutiquement acceptables, destinée à être utilisée dans la prophylaxie et le traitement d'une affection thrombotique.

2. 5-amino-2,3-dihydro-1,4-phtalazinedione ou l'un de ses sels, hydrates ou solvates pharmaceutiquement acceptables, destinée à être utilisée selon la revendication 1, le sel pharmaceutiquement acceptable étant le sel de sodium de 5-amino-2,3-dihydro-1,4-phtalazinedione.

3. 5-amino-2,3-dihydro-1,4-phtalazinedione ou l'un de ses sels pharmaceutiquement acceptables, d'hydrates ou de solvates, destinés à l'utilisation selon la revendication 2, le sel de sodium de la 5-amino-2,3-dihydro-1,4-phtalazinedione se présentant sous la forme de l'un des polymorphes cristallins anhydres I, Il ou III, **caractérisés par** des valeurs cristallographiques déterminées à l'aide de diffractogrammes de poudre aux rayons X :
valeurs d : 13,5 ; 6,9 ; 5,2 ; 4,6 ; 3,9 ; 3,5 ; 3,4 ; 3,3 ; 3,1 ; 3,0 et/ou
valeurs 2-thêta : 6,5 ; 12,7 ; 16,9 ; 19,3 ; 22,8 ; 25,8 ; 26,6 ; 27,2 ; 28,7 ; 30,3 pour la forme I,
valeurs d : 12,9 ; 7,9 ; 7,1 ; 6,5 ; 5,3 ; 4,0 ; 3,7 ; 3,6 ; 3,3 ; 3,2 et/ou
valeurs 2-théta : 6,8 ; 11,2 ; 12,5 ; 13,7 ; 16,7 ; 22,4 ; 24,3 ; 24,9 ; 27,2 ; 27,8 pour la forme Il ainsi que
valeurs d : 13,131 ; 7,987 ; 7,186 ; 6,566 ; 6,512 ; 5,372 ; 3,994 ; 3,662 ; 3,406 ; 3,288 ; 3,283 ; 3,222 ; 3,215 ; 3,127 ; 2,889 et/ou
valeurs 2-théta : 6,73 ; 11,07 ; 12,31 ; 13,48 ; 13,59 ; 16,49 ; 22,24 ; 24,29 ; 26,14 ; 27,10 ; 27,14 ; 27,67 ; 27,72 ; 28,52 ; 30,93 pour la forme III.

4. 5-amino-2,3-dihydro-1,4-phtalazinedione ou l'un de ses sels, hydrates ou solvates pharmaceutiquement acceptables, destinée à être utilisée selon l'une des revendications 2 ou 3, la dose journalière pour un patient qui en a besoin étant comprise entre 100 mg et 1 000 mg.

5. 5-amino-2,3-dihydro-1,4-phtalazinedione ou l'un de ses sels, hydrates ou solvates pharmaceutiquement acceptables, destinée à être utilisée selon l'une des revendications 1 à 4, la maladie thrombotique étant provoquée par une infection virale.

6. 5-amino-2,3-dihydro-1,4-phtalazinedione ou l'un de ses sels, hydrates ou solvates pharmaceutiquement acceptables, destinée à être utilisée selon la revendication 5, la maladie thrombotique étant provoquée par une infection par SARS-CoV-2.

7. 5-amino-2,3-dihydro-1,4-phtalazinedione ou l'un de ses sels pharmaceutiquement acceptables, destinée à être utilisée selon l'une des revendications 1 à 4, la maladie thrombotique étant choisie parmi un groupe comprenant la thrombose veineuse, la thrombose veineuse profonde, le syndrome de Paget-Schroetter, le syndrome de Budd-Chiari, la thrombose de la veine porte, la thrombose veineuse rénale, la thrombose des sinus cérébraux, la thrombose de la veine jugulaire, la thrombose du sinus caverneux, la thrombophlébite, la thrombose périnatale, la phlegmatia alba dolens, la thrombose artérielle, l'artériopathie oblitérante périphérique, l'ischémie aiguë des membres, la thrombose de l'artère hépatique, la coagulation intravasculaire disséminée, les troubles de la coagulation associés à des affections septiques et la microangiopathie thrombotique.

8. Composition pharmaceutique contenant de 5-amino-2,3-dihydro-1,4-phtalazinedione ou l'un de ses sels, hydrates ou solvates pharmaceutiquement acceptables, ainsi qu'au moins un excipient pharmaceutiquement acceptable, destinée à être utilisée dans la prophylaxie ou le traitement d'une affection thrombotique.

9. Composition pharmaceutique destinée à être utilisée selon la revendication 8, ladite composition pharmaceutique étant destinée à une administration par voie inhalatoire, orale, parentérale, intrapéritonéale, intraveineuse, intra-artérielle, intramusculaire, topique, transdermique, sous-cutanée, intradermique, sublinguale, conjonctivale, intravaginale, rectale, intrathécale, pharyngée ou nasale, ou pour une administration par intubation.

10. Composition pharmaceutique destinée à être utilisée selon l'une des revendications 8 ou 9, ledit au moins un excipient pharmaceutiquement acceptable étant choisi parmi un groupe comprenant véhicules, liants, colorants, tampons, conservateurs, antioxydants, revêtements, édulcorants, épaississants, régulateurs de pH, régulateurs d'acidité, agents acidifiants, solvants, agents isotonisants, agents désintégrants, agents glissants, lubrifiants, émulsifiants, agents solubilisants, stabilisants, diluants, agents anti-agglomérants, sorbants, promoteurs de perméation, agents moussants,
agents antimoussants, agents opacifiants, agents de retannage, promoteurs de consistance, hydrotropes ainsi que des arômes et des substances aromatiques.

11. 5-amino-2,3-dihydro-1,4-phtalazinedione ou l'un de ses sels pharmaceutiquement acceptables, d'hydrates ou de solvates, destinée à être utilisée selon la revendication 1, ou une composition pharmaceutique destinée à être utilisée selon la revendication 8, la forme d'administration par voie orale de ladite substance ou de ladite composition pharmaceutique se présentant sous forme de comprimés, de comprimés sublinguaux, de gélules en gélatine molle, de gélules en gélatine dure, de comprimés ou pilules revêtis de sucre, de poudres ou de granulés, de jus, de sirops, de gouttes, de tisanes, de solutions ou de suspensions dans des liquides aqueux ou non aqueux, de mousses comestibles, de pastilles à sucer ou dans des émulsions huile-dans-eau ou eau-dans-huile.

12. 5-amino-2,3-dihydro-1,4-phtalazinedione ou l'un de ses sels pharmaceutiquement acceptables, d'hydrates ou de solvates, destinée à être utilisée selon la revendication 1, ou une composition pharmaceutique destinée à être utilisée selon la revendication 8, ladite substance ou ladite composition pharmaceutique étant administrée par inhalation à l'aide d'un nébuliseur à maille vibrante, d'un inhalateur-doseur, d'un nébuliseur à jet ou d'un inhalateur à poudre sèche.

13. 5-amino-2,3-dihydro-1,4-phtalazinedione ou l'un de ses sels, hydrates ou solvates pharmaceutiquement acceptables, destinée à être utilisée selon la revendication 1, ou une composition pharmaceutique destinée à être utilisée selon la revendication 8, ladite substance ou ladite composition pharmaceutique étant formulée sous forme de préparation à action retardée.

14. 5-amino-2,3-dihydro-1,4-phtalazinedione ou l'un de ses sels, hydrates ou solvates pharmaceutiquement acceptables, destinée à être utilisée selon la revendication 1, ou une composition pharmaceutique destinée à être utilisée selon la revendication 8, ladite substance ou ladite composition pharmaceutique étant administrée sous forme de liposomes, de micelles, de vésicules multilamellaires ou d'un complexe de cyclodextrine.

15. 5-amino-2,3-dihydro-1,4-phtalazinedione ou l'un de ses sels, hydrates ou solvates pharmaceutiquement acceptables, destinée à être utilisée selon la revendication 1, ou une composition pharmaceutique destinée à être utilisée selon la revendication 8, ladite substance ou ladite composition pharmaceutique étant appliquée par voie topique sous forme de crèmes, d'émulsions, de lotions, de gels, d'hydrogels, de pâtes, de poudres, de pommades, de liniments, de films, de liposomes, de patchs cutanés, de patchs transdermiques, de sprays transdermiques ou de suspensions.
